(19) Europäisches Patentamt
European Patent Office
Office européen des brevets

(11) EP 0 557 203 B2

(12) # NOUVEAU FASCICULE DE BREVET EUROPEEN

(45) Date de publication et mention de la décision concernant l'opposition:
**02.12.1998 Bulletin 1998/49**

(45) Mention de la délivrance du brevet:
**10.07.1996 Bulletin 1996/28**

(21) Numéro de dépôt: **93400433.4**

(22) Date de dépôt: **19.02.1993**

(51) Int. Cl.$^6$: **A61K 7/08**, A61K 7/13, A61K 7/135, A61K 7/06

(54) **Composition cosmétique à base d'agents tensio-actifs non-ioniques et de polymères substantifs cationiques ou amphotères et son utilisation comme support de teinture ou de décoloration**

Kosmetische Zusammensetzung enthaltend nichtionische Tensio und kationische oder amphotere Polymers sowie ihre Verwendung wie Träger in Haarfärbemittel oder Haarbleichungsmittel

Cosmetic composition containing nonionic surfactants and cationic or amphoteric substantive polymers and its use as carrier in dyeing or bleaching compositions

(84) Etats contractants désignés:
**AT BE CH DE DK ES FR GB GR IE IT LI NL PT SE**

(30) Priorité: **21.02.1992 FR 9202051**

(43) Date de publication de la demande:
**25.08.1993 Bulletin 1993/34**

(73) Titulaire: **L'OREAL**
**75008 Paris (FR)**

(72) Inventeurs:
- **Millequant, Jean-Marie**
**F-94100 Saint-Maur (FR)**
- **Boudy, François**
**F-75014 Paris (FR)**

(74) Mandataire: **Casalonga, Axel et al**
**BUREAU D.A. CASALONGA - JOSSE**
**Morassistrasse 8**
**80469 München (DE)**

(56) Documents cités:

| | |
|---|---|
| EP-A- 0 188 216 | EP-A- 0 216 334 |
| EP-A- 0 295 474 | EP-A- 0 366 542 |
| EP-A- 0 376 078 | EP-A- 0 424 261 |
| EP-A- 0 428 441 | DD-A- 221 922 |
| DD-A- 271 219 | DE-C- 2 936 934 |

Remarques:
Le dossier contient des informations techniques présentées postérieurement au dépôt de la demande et ne figurant pas dans le présent fascicule.

EP 0 557 203 B2

**Description**

La présente invention a pour objet une nouvelle composition cosmétique à base d'agents tensio-actifs non-ioniques et de polymères substantifs cationiques ou amphotères, l'utilisation de cette composition pour la préparation de compositions de teinture ou de décoloration des fibres kératiniques et les procédés de teinture ou de décoloration mettant en oeuvre de telles compositions.

On connaît des compositions renfermant un ou plusieurs agents tensio-actifs non-ioniques dont en particulier des composés non-ioniques polyoxyéthylénés ou polyglycérolés.

On sait également que les polymères cationiques permettent d'améliorer l'état cosmétique des cheveux, en particulier le démêlage. On cherche de ce fait à utiliser dans les compositions cosmétiques contenant des agents tensioactifs non-ioniques utilisées à titre de support de coloration et de décoloration, des polymères cationiques en vue de conférer aux fibres kératiniques colorées et décolorées de bonnes propriétés cosmétiques, notamment de démêlage.

On a cependant constaté que certains polymères cationiques posaient des problèmes de compatibilité avec les supports non-ioniques, se manifestant par une séparation de phases au cours du stockage, une modification de la consistance et des propriétés moussantes.

Ce problème avait été réglé en particulier dans FR-A-2.502.949 dans le cas de compositions tinctoriales d'oxydation mettant en oeuvre une solution oxydante, par la mise en oeuvre d'un procédé consistant à stocker séparément, d'une part, le polymère cationique dans la solution oxydante contenant notamment le peroxyde d'hydrogène et, d'autre part, la composition tinctoriale contenant les colorants et les agents tensio-actifs non-ioniques. Le mélange des deux compositions s'effectuant au moment de l'emploi. Le documents EP-A-0 295 474 et EP-A-0 366 542 décrivent des compositions pour les cheveux.

La demanderesse a découvert, ce qui fait l'objet de l'invention, qu'il était possible d'éviter la séparation de la composition utilisée comme support, en particulier de teinture ou de décoloration, en plusieurs phases ou la précipitation des ingrédients du support en introduisant dans une composition contenant des agents tensio-actifs non-ioniques particuliers, des polymères substantifs cationiques ou amphotères réputés incompatibles avec le milieu non-ionique de ladite composition pour obtenir une composition stable à la température et au cours du temps.

L'invention a donc pour objet une composition cosmétique contenant dans un milieu cosmetiquement acceptable, un mélange d'agents tensio-actifs non-ioniques particulier et des polymères substantifs de type cationique ou amphotère.

Un autre objet de l'invention est constitué par l'utilisation de cette composition comme support dans des compositions tinctoriales ou de décoloration.

L'invention a également pour objet les compositions tinctoriales ou de décoloration ainsi obtenues ainsi que leur utilisation pour la coloration et la décoloration des cheveux.

D'autres objets de l'invention apparaîtront à la lecture de la description et des exemples qui suivent.

La composition conforme à l'invention est essentiellement caractérisée par le fait qu'elle contient dans un milieu cosmétiquement acceptable :

a) 14 à 50%, de préférence 20 à 50% en poids d'un mélange d'agents tensio-actifs non-ioniques choisis parmi les alcools gras, les alcools gras oxyéthylénés et/ou oxypropylénés et/ou polyglycérolés, linéaires ou ramifiés pouvant comporter des motifs aromatiques, le mélange comprenant au moins un agent tensio-actif A ayant un HLB au sens de Griffin supérieur ou égal à 14, cet agent tensio-actif étant présent dans une quantité pondérale égale à [A] et au moins un agent tensio-actif non-ionique B dont le HLB au sens de Griffin est supérieur ou égal à 1 et inférieur à 10, présent dans une quantité pondérale [B], le rapport

$$R = \frac{\Sigma(nC_A \times [A])}{\Sigma(nC_B \times [B])} = \frac{\text{Somme des produits } nC_A \times [A]}{\text{Somme des produits } nC_B \times [B]}$$

dans lequel :

$nC_A$ et $nC_B$ désignent respectivement le nombre d'atomes de carbone de la chaîne grasse des tensio-actifs A et B,
étant tel que :
$0{,}5 \leqq R \leqq 1{,}6$, plus de 50% et de préférence au moins 52% des agents tensio-actifs non-ioniques présents vérifiant l'inégalité pour R;

b) 0,05 à 10% en poids d'un polymère substantif cationique ou amphotère, ladite composition étant stable à température ambiante et a un pH égal ou supérieur à 5,5.

Les agents tensio-actifs non-ioniques utilisables conformément à l'invention du type A et présentant un HLB au sens de Griffin supérieur ou égal à 14, sont choisis de préférence parmi les alcools gras dont la chaîne grasse comporte un nombre d'atomes de carbone de 12 à 30 ($12 \leqq nC_A \leqq 30$) et les agents tensio-actifs de type B ayant un HLB au sens de Griffin compris entre 1 et 10, sont choisis de préférence parmi les alcools gras dont la chaîne grasse comporte un nombre d'atomes de carbone de 10 à 50 ($10 \leqq nC_B \leqq 50$).

Parmi les agents tensio-actifs de type A, on peut citer plus particulièrement :

- l'alcool oléocétylique oxyéthyléné à 30 moles d'oxyde d'éthylène $nC_A$ = 17 (valeur moyenne)
  HLB = 16,5
- l'alcool laurique ($C_{12}$-$C_{14}$/55 - 45%) oxyéthyléné à 12 moles d'oxyde d'éthylène
  $nC_A$ = 12,5
  HLB = 14
- l'alcool laurique oxyéthyléné à 23 moles d'oxyde d'éthylène
  $nC_A$ = 12
  HLB = 16,6
- l'alcool cétylstéarylique oxyéthyléné à 15 moles d'oxyde d'éthylène
  $nC_A$ = 17
  HLB = 14,5
- l'alcool stéarylique oxyéthyléné à 100 moles d'oxyde d'éthylène
  $nC_A$ = 18
  HLB = 18,8
- l'alcool isostéarylique oxyéthyléné à 20 moles d'oxyde d'éthylène
  $nC_A$ = 18
  HLB = 14,9
- l'alcool béhénique oxyéthyléné à 20 moles d'oxyde d'éthylène
  $nC_A$ = 22
  HLB = 16,5
- l'alcool primaire en $C_{30}$ oxyéthyléné à 40 moles d'oxyde d'éthylène
  $nC_A$ = 30
  HLB = 16,1
- les octylphénols oxyéthylénés de 11 à 50 moles d'oxyde d'éthylène, les nonylphénols oxyéthylénés 12 à 50 moles d'oxyde d'éthylène.

Parmi les alcools gras de type B, on peut citer plus particulièrement :

- l'alcool décylique oxyéthyléné à 3,5 moles d'oxyde d'éthylène
  $nC_B$ = 10,4
  HLB = 8,5
- l'alcool oléique à 70% de $C_{18}$
  $nC_B$ = 17,5
  HLB = 1
- l'alcool laurique
  $nC_B$ = 12
  HLB = 1
- l'alcool cétylstéarylique (50/50)
  $nC_B$ = 17
  HLB = 1
- l'alcool isostéarylique oxyéthyléné à 2 moles d'oxyde d'éthylène
  $nC_B$ = 18
  HLB = 6,5
- l'alcool oléique oxyéthyléné à 3 moles d'oxyde d'éthylène
  $nC_B$ = 18
  HLB = 6,5
- l'alcool laurique oxyéthyléné à 4 moles d'oxyde d'éthylène
  $nC_B$ = 12
  HLB = 9,7
- l'alcool laurique oxypropyléné à 3 moles d'oxyde de propylène
  $nC_B$ = 12

HLB peu différent de 1

- l'alcool primaire en $C_{50}$ oxyéthylénéà 4 moles d'oxyde d'éthylène
  $nC_B$ = 50
  HLB = 4
- l'alcool oléique polyglycérolé à 2 moles de glycérol
  $nC_B$ = 18
  HLB = 7,1
- des octylphénols oxyéthylénés à moins de 4,5 moles d'oxyde d'éthylène
- des nonylphénols oxyéthylénés à moins de 5 moles d'oxyde d'éthylène.

La valeur HLB selon Griffin est définie dans J. Soc. Cosm. Chem. 1954 (volume 5), pages 249-256.

La composition conforme à l'invention peut renfermer dans une proportion inférieure à 50%, en particulier égale ou inférieure à 48% de la totalité des agents tensio-actifs présents, des agents tensio-actifs non-ioniques présentant un HLB intermédiaire selon Griffin compris entre 10 et 14.

Parmi ces agents tensio-actifs, on peut citer l'alcool décylique oxyéthyléné à 5,5 moles d'oxyde d'éthylène (HLB = 11,5), le nonyl-phénol oxyéthyléné à 6 moles d'oxyde d'éthylène (HLB = 11), l'acool primaire en $C_{30}$ oxyéthyléné à 10 moles d'oxyde d'éthylène (HLB=10).

Le caractère substantif des polymères cationiques ou amphotères utilisés conformément à l'invention, est déterminé par le test à l'aide du colorant acide Red 80, selon RICHARD J. CRAWFORD, Journal of the Society of Cosmetic Chemists, 1980-31-(5)-pages 273 à 278.

Ces polymères sont entre autres des polymères qui sont incompatibles avec un support non-ionique ne répondant pas à la définition conforme à l'invention.

Le caractère incompatible peut être déterminé en particulier en introduisant 1% des matières actives de ce polymère substantif dans un milieu non-ionique tel que celui décrit dans le FR 2 502 949. L'apparition d'un troupe plus ou moins accentué, éventuellement même d'un précipité et/ou la séparation de la composition en deux phases, est considérée comme le caractère d'incompatibilité.

Les polymères substantifs sont choisis en particulier parmi des polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les protéines quaternisées, les polymères de la famille des polyamines, polyaminoamides, polyammonium quaternaires et les polysiloxanes cationiques.

**A.** Les protéines quaternisées sont en particulier des polypeptides modifiés chimquement et portant en bout de chaîne ou greffés sur celle-ci, des groupements ammonium quaternaires. Parmi ces protéines, on peut citer notamment :

- les hydrolysats de collagène portant des groupements triéthyl-ammonium tels que les produits vendus sous la dénomination "QUAT-PRO E" par la Société MAYBROOK et dénommés dans le dictionnaire CTFA "Triéthonium Hydrolyzed Collagen Ethosulfate";
- les hydrolysats de collagène portant des groupements chlorure de triméthylammonium ou de diméthylstéarylammonium vendus sous la dénomination de "QUAT-PRO S" par la Société MAYBROOK et dénommés dans le dictionnaire CTFA "Steartrimonium Hydrolyzed Collagen";
- les hydrolysats de protéines animales portant des groupements diméthylbenzylammonium tels que les produits vendus sous la dénomination "CROTEIN BTA" par la Société CRODA et dénommés dans le dictionnaire CTFA "Benzyltrimonium Hydrolyzed Animal Protein";
- les hydrolysats de protéines portant sur la chaîne polypeptidique des groupements ammonium quaternaires comportant au moins un radical alkyle ayant de 1 à 18 atomes de carbone. Parmi ces hydrolysats de protéines, on peut citer entre autres :
- le CROQUAT L dont la chaîne polypeptidique a un poids moléculaire moyen d'environ 2500 et dont le groupement ammonium quaternaire comporte un groupement alkyle en $C_{12}$;
- le CROQUAT M dont la chaîne polypeptidique a un poids moléculaire moyen d'environ 2500 et dont le groupement ammonium quaternaire comporte un groupement alkyle en $C_{10}$-$C_{18}$;
- le CROQUAT S dont la chaîne polypeptidique a un poids moléculaire moyen d'environ 2700 et dont le groupement ammonium quaternaire comporte un groupement alkyle en $C_{18}$;
- le CROQUAT Q dont la chaîne polypeptidique a un poids moléculaire moyen de l'ordre de 12 000 et dont le groupement ammonium quaternaire comporte au moins un groupe alkyle ayant de 1 à 18 atomes de carbone;
- une protéine quaternisée végétale de soja vendue sous la dénomination CROQUAT SOYA.

Ces différents produits sont vendus par la Société CRODA.

- une protéine quaternisée résultant de la condensation de la cocamidopropyldiméthylamine sur une protéine animale hydrolysée, dénommée, dans le dictionnaire CTFA édition 1991, quaternium 76 Hydrolysed Collagen vendue sous la dénomination LEXEIN QX 3000 par la Société INOLEX.

**B.** Les polymères de la famille des polyamines, polyaminoamides ou polyammonium quaternaires, utilisables conformément à la présente invention, sont décrits en particulier dans les brevets français de la demanderesse n°2.505.348, 2.542.997 et 2.598.613.

Parmi ces polymères, on peut citer :

(1) Les copolymères vinylpyrrolidone/acrylate ou méthacrylate de dialkylaminoalkyle quaternisés ou non, tels que les produits vendus sous la dénomination "GAFQUAT" par la Société GAF CORPORATION comme par exemple "GAFQUAT 734 ou 755", ou bien les produits dénommés "COPOLYMER 845, 958 et 937". Ces polymères sont décrits en détail dans les brevets français 2.077.143 et 2.393.573, et le copolymère vinylpyrrolidone/méthacrylamide de diméthylaminopropyle quaternisé tel que le produit vendu sous la dénomination "GAFQUAT HS100" par la Société GAF.
(2) Les dérivés d'éthers de cellulose comportant des groupements ammonium quaternaires décrits dans le brevet français 1.492.597 et en particulier les polymères commercialisés sous les dénominations "JR" (JR 400, JR 125, JR 30M) ou "LR" (LR 400, LR 30M) par la Société UNION CARBIDE CORPORATION. Les polymères sont également définis dans le dictionnaire CTFA comme des ammonium quaternaires d'hydroxyéthylcellulose ayant réagi avec un époxyde substitué par un groupement triméthylammonium. (3) Les dérivés de cellulose cationiques tels que les copolymères de cellulose ou les dérivés de cellulose greffés par un monomère hydrosoluble d'ammonium quaternaire et décrits plus en détail dans le brevet américain 4.131.576 tels que les hydroxyalkylcelluloses comme les hydroxyméthyl-, hydroxyéthyl- ou hydroxypropylcellulose greffées par un sel de méthacryloyléthyltriméthylammonium méthacrylamidopropyltriméthylammonium, diméthyldiallylammonium.

Les produits commercialisés répondant à cette définition sont plus particulièrement les produits vendus sous les dénominations "CELQUAT L 200" et "CELQUAT H 100" par la Société NATIONAL STARCH.
(4) Les polysaccharides quaternisés décrits plus particulièrement dans les brevets américains 3.589.578 et 4.031.307 tel que le produit commercialisé sous la dénomination "JAGUAR C 13 S" vendu par la Société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont décrits dans les brevets français 2.162.025 et 2.280.361.
(6) Les polyaminoamides solubles dans l'eau préparés en particulier par polycondensation d'un composé acide. avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mode par groupement amine du polyaminoamide.

Ces polyaminopolyamides peuvent être alcoylés ou s'ils comportent une ou plusieurs fonctions amines tertiaires, quaternisés. De tels polymères sont décrits en particulier dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont décrits dans le brevet français 1.583.363.

Parmi ces dérivés, on peut citer plus particulièrement les polymères acide adipique/diméthylaminohydroxypropyl/diéthylène triamine vendus sous la dénomination "CARTARETINE F, $F_4$ ou $F_8$" par la Société SANDOZ.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont décrits en

particulier dans les brevets américains 3.227.615 et 2.961.347.

Des polymères de ce type sont en particulier commercialisés sous la dénomination "HERCOSETT 57" par la Société HERCULES INCORPORATED ou bien sous la dénomination de "PD 170" ou "DELSETTE 101" par la Société HERCULES dans le cas du copolymère d'acide adipique/époxypropyl/diéthylène-triamine.

(9) Les cyclopolymères ayant un poids moléculaire de 20 000 à 3 000 000 tels que les homopolymères comportant comme constituant principal de la chaîne des motifs répondant aux formules (II) ou (II') :

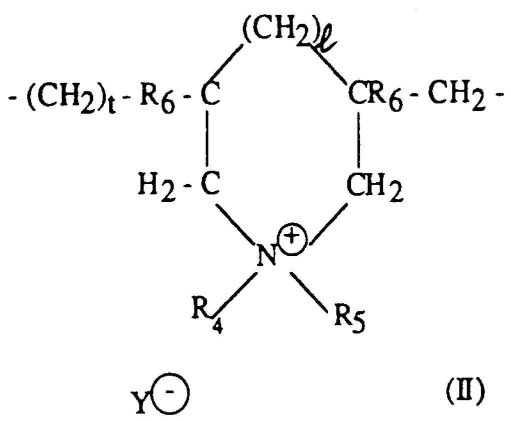

(II)

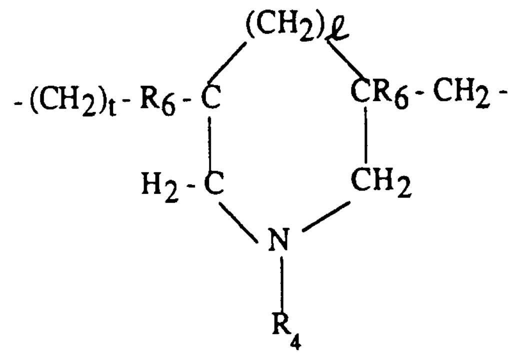

(II')

$\ell$ et t sont égaux à 0 ou 1, et la somme $\ell$ + t = 1;

$R_6$ désigne hydrogène ou méthyle;

$R_4$ et $R_5$ désignent, indépendamment l'un de l'autre, un groupement alkyle ayant de 1 à 22 atomes de carbone, un groupement hydroxyalkyle dans lequel le groupement alkyle a de préférence 1 à 5 atomes de carbone, un groupement amidoalkyle inférieur et où $R_4$ et $R_5$ peuvent désigner conjointement avec l'atome d'azote auquel ils sont rattachés, des groupements hétérocycliques tels que pipéridinyle ou morpholinyle, ainsi que les copolymères comportant des unités de formules (II) ou (II') et des unités dérivés d'acrylamide ou de diacétone acrylamide;

$Y^{\ominus}$ est un anion tel que bromure, chlorure, acétate, borate, citrate, tartrate, bisulfate, bisulfite, sulfate, phosphate.

Parmi les polymères définis ci-dessus, on peut citer plus particulièrement l'homopolymère de chlorure de diméthyldiallylammonium vendu sous la dénomination "MERQUAT 100", ayant un poids moléculaire inférieur à 100 000, le copolymère de chlorure de diméthyldiallylammonium et d'acrylamide ayant un poids moléculaire supérieur à 500 000 et vendu sous la dénomination "MERQUAT 550" par la Société MERCK.

Ces polymères sont décrits plus particulièrement dans le brevet français 2.080.759, et son certificat d'addition n°2.190.406.

(10) Le polymère de polyammonium quatenaire contenant des motifs récurrents répondant à la formule :

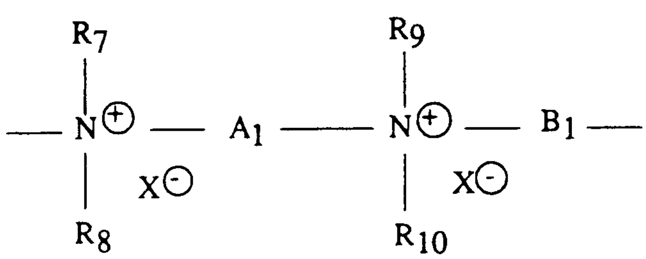

(III)

dans laquelle $R_7$ et $R_8$, $R_9$ et $R_{10}$ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien $R_7$ et $R_8$ et $R_9$ et $R_{10}$ ensemble ou séparément, constituent avec les atomes d'azote auxquels ils sont rattachés, des hétérocycles contenant éventuellement un second hétéroatome autre que l'azote ou bien $R_7$, $R_8$, $R_9$ et $R_{10}$ représentent un radical alkyle en $C_1$-$C_6$ linéaire ou ramifié substitué par un groupement nitrile, ester,

acyle, amide ou

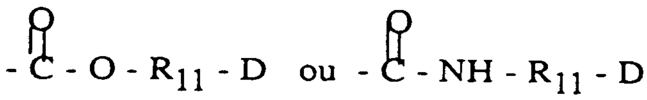

où $R_{11}$ est un alkylène et D un groupement ammonium quaternaire.

$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

$X^{\ominus}$ désigne un anion dérivé d'un acide minéral ou organique.

$A_1$ et $R_7$ et $R_9$ peuvent former avec les deux atomes d'azote auxquels ils sont rattachés, un cycle pipérazinique; en outre, si $A_1$ désigne un radical alkylène ou hydroxyalkylène linéaire ou ramifié, saturé ou insaturé, $B_1$ peut également désigner un groupement $(CH_2)_n - CO - D - OC - (CH_2)_n$ - dans lequel D désigne :

a) un reste de glycol de formule : - O - Z - O - où Z désigne un radical hydrocarboné linéaire ou ramifié ou un groupement répondant aux formules :

$$-[CH_2 - CH_2 - O]_x - CH_2 - CH_2 -$$

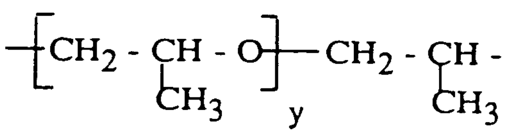

où x et y désignent un nombre entier de 1 à 4, représentant un degré de polymérisation défini et unique ou un nombre quelconque de 1 à 4 représentant un degré de polymérisation moyen;

b) un reste de diamine bis-secondaire tel qu'un dérivé de pipérazine;

c) un reste de diamine bis-primaire de formule :

$$- NH - Y - NH -$$

où Y désigne un radical hydrocarboné linéaire ou ramifié, ou bien le radical bivalent

$$- CH_2 - CH_2 - S - S - CH_2 - CH_2 -$$

d) un groupement uréylène de formule :

$$- NH - CO - NH - ;$$

$X^{\ominus}$ est un anion tel que chlorure ou bromure.

Ces polymères ont une masse moléculaire généralement comprise entre 1000 et 100 000.

Des polymères de ce type sont décrits en particulier dans les brevets français 2 320 330, 2 270 846, 2 316 271, 2 336 434, et 2 413 907 et les brevets US-A-2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

(11) Les polymères de polyammonium quaternaires constitués de motifs de formule :

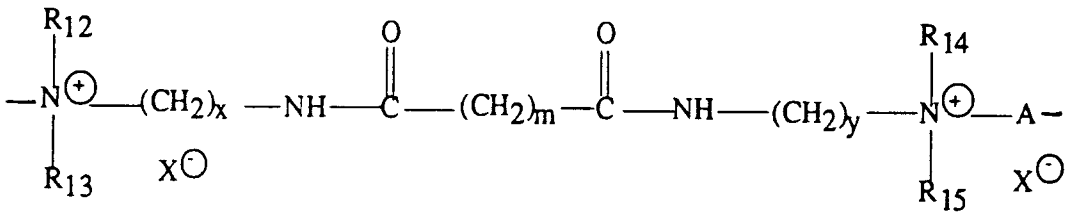 (IV)

dans laquelle :

$R_{12}$, $R_{13}$, $R_{14}$ et $R_{15}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à O ou un nombre entier compris entre 1 et 6, sous réserve que $R_{12}$, $R_{13}$, $R_{14}$ et $R_{15}$ ne représentent pas simultanément un atome d'hydrogène;
x et y, identiques ou différents, sont des nombres entiers compris entre 1 et 6;
m est égal à O ou à un nombre entier compris entre 1 et 34;
x désigne un atome d'halogène;
A désigne un radical d'un dihalogénure et représente de préférence

$$-CH_2-CH_2-O-CH_2-CH_2-$$

De tels composés sont décrits plus en détail dans la demande EP-A-122 24.

On peut par exemple citer parmi ceux-ci, les produits "MIRAPOL A 15", "MIRAPOL AD1", "MIRAPOL AZ1", "MIRAPOL 175", vendus par la Société MIRANOL.

(12) Les homopolymères ou copolymères dérivés d'esters ou d'amides acrylique ou méthacrylique et comportant les motifs :

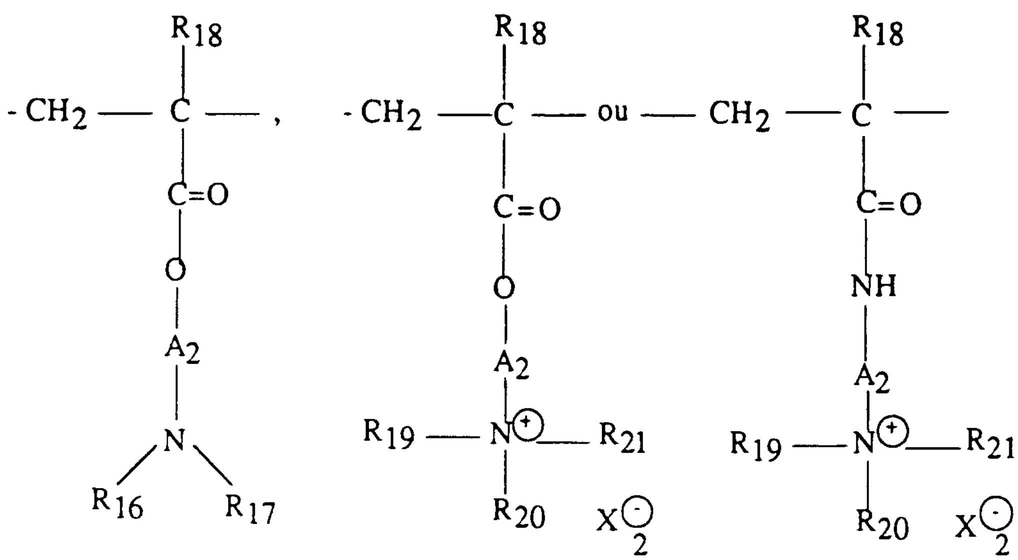
, ou (V)

dans lesquels :

$R_{18}$ désigne H ou $CH_3$;
$A_2$ est un groupe alkyle linéaire ou ramifié de 1 à 6 atomes de carbone ou un groupe hydroxyalkyle de 1 à 4 atomes de carbone;
$R_{19}$, $R_{20}$, $R_{21}$, identiques ou différents, représentent un groupe alkyle ayant de 1 à 18 atomes de carbone ou un radical benzyle;

$R_{16}$ et $R_{17}$ représentent hydrogène ou un groupe alkyle ayant de 1 à 6 atomes de carbone;
$X^{\ominus}{}_2$ désigne un anion méthosulfate ou un halogénure tel que chlorure ou bromure.

Le ou les comonomères utilisables appartiennent à la famille des acrylamide, méthacrylamide, diacétone acrylamide, acrylamide et méthacrylamide substitués à l'azote par des alkyles inférieurs, des actes acrylique ou méthacrylique ou leurs esters, la vinylpyrrolidone, des esters vinyliques.

Parmi ces composés, on peut crier le copolymère d'acrylamide et de diméthylaminoèthyl méthacrylate quaternisé au sulfate de diméthyle et vendu sous la dénomination "HERCOFLOC" par la Société HERCULES, le copolymère d'acrylamide et de chlorure de méthacryloyloxyéthyltriméthylammonium - décrit dans la demande de brevet EP-A-80976 - et vendu sous la dénomination "BINA QAT P100" par la Société CIBA GEIGY, ou encore le poly(chlorure de méthacrylamidopropyltriméthylammonium) vendu sous la dénomination "POLYMAPTAC" par la Société TEXACO CHEMICALS, le méthacryloyloxyéthyltriméthylammonium méthosulfate et son copolymère avec l'acrylamide vendus sous la dénomination "RETEN" par la Société HERCULES. (13) Les polymères quaternaires de vinylpyrrolidone et de vinylimidazole tel que par exemple les produits commercialisés sous les dénominations "LUVIQUAT FC 905, FC 550 et FC 370" par la Société BASF. (14) Les polyamines comme le Polyquart H vendu par HENKEL, référencé sous le nom de "POLYETHYLENEGLYCOL (15) TALLOW POLYAMINE" dans le dictionnaire CTFA.

D'autres polymères substantifs utilisables conformément à l'invention sont des polyalkylèneimines, en particulier des polyéthylèneimines, des polymères contenant des motifs vinylpyridine ou vinylpyridinium, des condensats de polyamines et d'épichlorhydrine, les polyuréylènes quaternaires et les dérivés de la chitine.

Les polysiloxanes cationiques sont choisis plus particulièrement parmi :

(1) les polymères de formule :

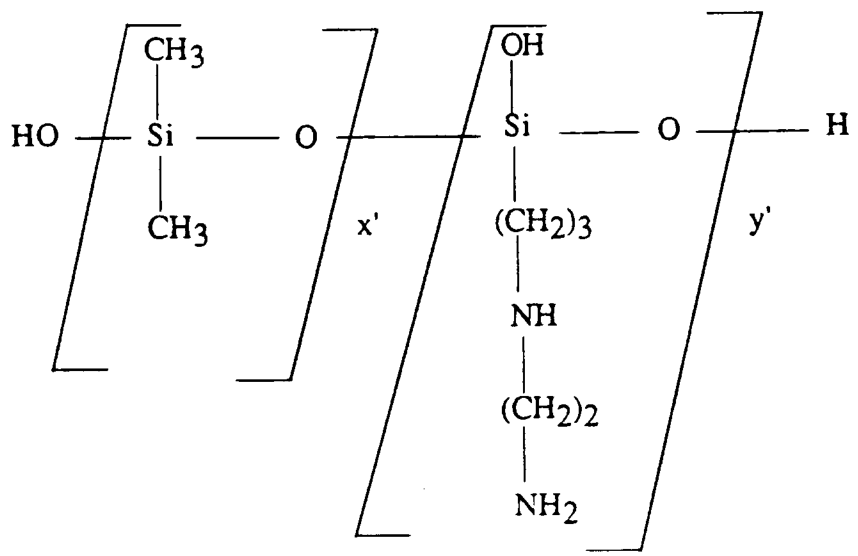

(VI)

dans laquelle x' et y' sont des nombres entiers dépendant du poids moléculaire qui est compris généralement entre 5000 et 10 000. Ces produits sont dénommés "Amodiméthicone" dans le dictionnaire CTFA.

(2) les polymères cationiques siliconés répondant à la formule :

$$R'_a G_{3-a}-Si(OSiG_2)_n-(OSi G_b R'_{2-b})_m-O-Si-G_{3-a}-R'_a \quad (VII)$$

dans laquelle :

G est un atome d'hydrogène, ou le groupement phényle, OH, un groupement alkyle en $C_1$-$C_8$ et de préférence méthyle;
a désigne 0 ou un nombre entier de 1 à 3 et de préférence 0;
b désigne 0 ou 1 et de préférence 1;
la somme (n+m) est un nombre entier de 1 à 2000 et de préférence de 50 à 150, n pouvant désigner un nombre de 0 à 1999 et de préférence de 49 à 149 et m pouvant désigner un nombre entier de 1 à 2000 et de préférence de 1 à 10;

R' est un radical monovalent de formule $C_qH_{2q}L$, dans laquelle q est un nombre de 2 à 8 et L est choisi parmi les groupements :

- $N(R'') - CH_2 - CH_2 - N(R'')_2$
- $N(R'')_2$
- $N^{\oplus}(R'')_3A^-$
- $N^{\oplus}(R'')H_2A^-$
- $N(R'')CH_2 - CH_2 - N^{\oplus} (R'')H_2\ A^-$ dans lesquelles R'' peut désigner hydrogène, phényle, benzyle, un radical hydrocarboné saturé monovalent et de préférence un radical alkyle ayant de 1 à 20 atomes de carbone et $A^-$représente un ion halogénure tel que chlorure, bromure, iodure ou fluorure.

Un produit particulièrement intéressant entrant dans cette définition est le polymère dénommé "triméthylsilylamodiméthicone" répondant à la formule :

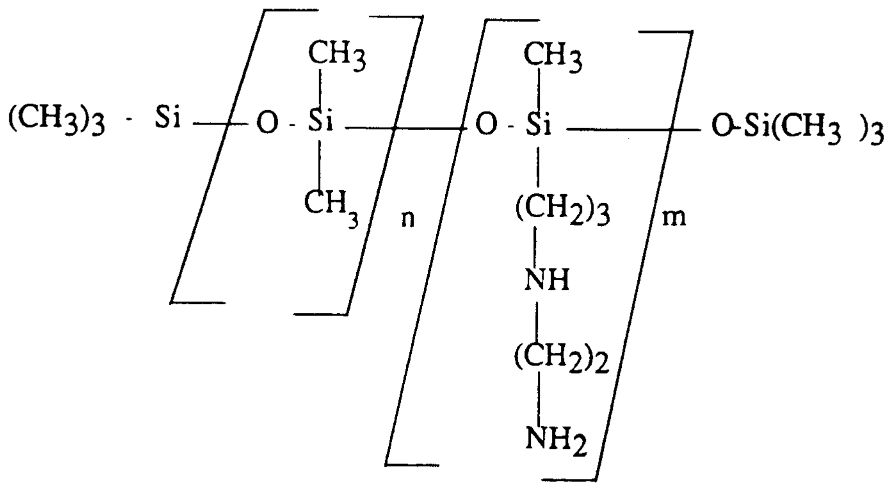

(VIII)

dans laquelle net mont les significations données ci-dessus pour x' et y' (formule VI). De tels polymères sont décrits dans la demande de brevet EP-A-95 238;

(3) les polymères cationiques siliconés répondant à la formule :

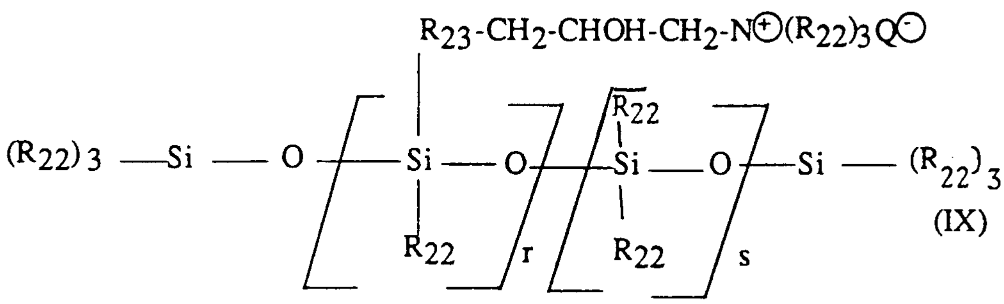

(IX)

dans laquelle :

$R_{22}$ désigne un radical hydrocarboné monovalent ayant de 1 à 18 atomes de carbone et en particulier un radical alkyle ou alcényle et de préférence méthyle;

$R_{23}$ désigne un radical hydrocarboné divalent, de préférence un radical alkylène en $C_1$-$C_{18}$ ou un radical alkylènoxy divalent en $C_1$-$C_{18}$ et de préférence en $C_1$-$C_8$;

$Q^{\ominus}$ est un ion halogénure, de préférence chlorure;

r représente une valeur statistique moyenne de 2 à 20 et de préférence de 2 à 8;

a représente une valeur statistique moyenne de 20 à 200 et de préférence de 20 à 50.

De tels polymères sont décrits plus particulièrement dans le brevet US-A-4 185 087.

Un polymère particulièrement préféré entrant dans cette classe est le polymère vendu par la Société UNION CARBIDE sous la dénomination "UCAR SILICONE ALE 56" qui se caractérise par un point éclair selon la norme ASTDM-93 de 60°C, une viscosité à la concentration de 35% de matière active et à 25°C de 0,011 Pa.s et par un indice de basicité totale de 0,24 meq/g.

Lorsque ces polymères siliconés sont mis en oeuvre, une forme de réalisation particulièrement intéressante est leur utilisation conjante avec des agents de surface non-ioniques, éventuellement des agents de surface cationiques. On peut utiliser par exemple dans les compositions conformément à l'invention, le produit commercial vendu sous la dénomination "EMULSION CATIONIQUE DC 929" par la Société DOW CORNING qui comprend l'amodiméthicone de formule (VI), un agent de surface cationique répondant à la formule :

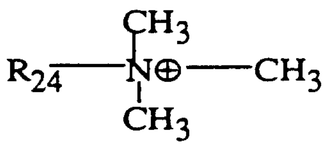 $Cl^-$ (X)

dans laquelle $R_{24}$ désigne un mélange de radicaux alcényle et/ou alcoyle ayant de 14 à 22 atomes de carbone dérivés des actes gras du suif, et un agent de surface non-ionique de formule :

$$C_{19}H_{19} - C_6H_4 - (OC_2H_4)_{10} - OH$$

connu sous la dénomination "NONOXYNOL 10".

Une autre composition utilise dans cette forme de réalisation de l'invention est la composition contenant le produit vendu sous la dénomination "DOW CORNING Q2 7224" par la Société DOW CORNING comportant en association le triméthylsilylamodiméthicone de formule (VIII), un agent de surface non-ionique de formule :

$$C_8H_{17} - C_6H_4 - (OCH_2CH_2)_n\ OH \text{ où } n = 40$$

dénommé encore octoxynol-40, un autre agent de surface non-ionique de formule :

$$C_{12}H_{25} - (OCH_2 - CH_2)_n\ OH \text{ où } n = 6$$

encore dénommé isolaureth-6 et du glycol.

Les polymères substantifs peuvent être choisis parmi les polymères amphotères tels que les polymères amphotères dérivés du chitosane ou les copolymères de diallyldialkylammonium et d'un monomère anionique.

(1) les polymères amphotères dérivés du chitosane sont choisis en particulier parmi les polymères comportant des motifs répondant aux formules :

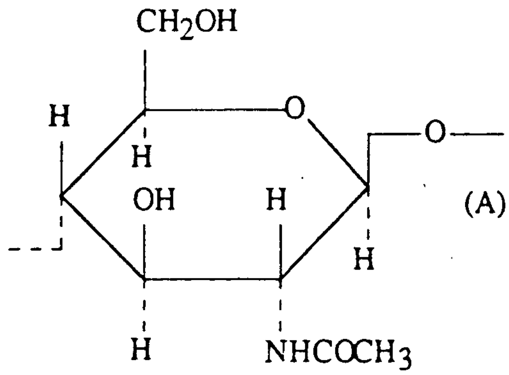
(A)

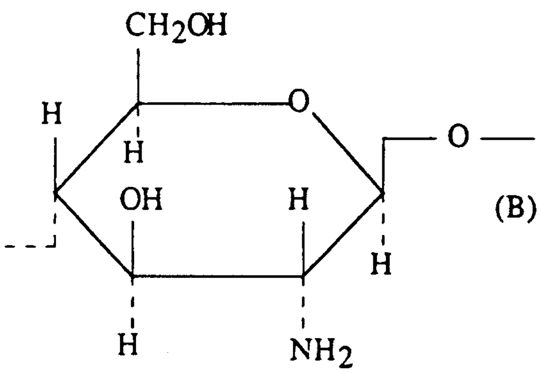
(B)

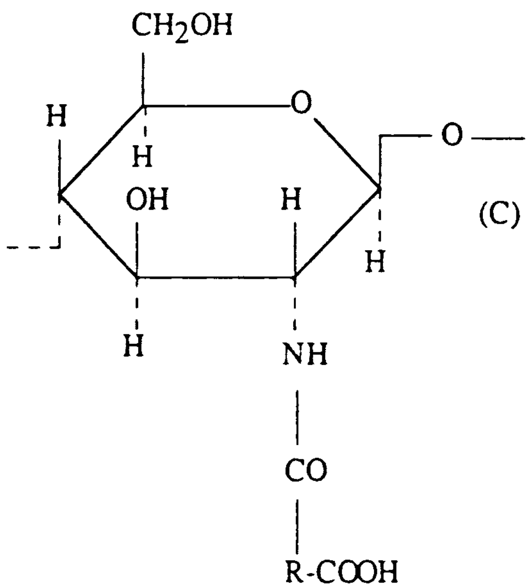
(C)

(XI)

Le motif (A) est présent dans des proportions de 0 à 30%, le motif (B) de 5 à 50%, le motif (C) de 30 à 90% en poids. R représente un groupement alkylène linéaire ou ramifié comportant de 2 à 5 atomes de carbone.

Le polymère préféré comporte de préférence 0 à 20% de motif (A), 40 à 50% de motif (B) et 40 à 50% de motif (C) dans lequel R désigne un radical alkylène et de préférence $-CH_2-CH_2-$.

De tels polymères sont décrits plus particulièrement dans le brevet FR-A-2.137.684.

(2) les polymères amphotères de diallyldialkylammonium et d'un monomère anionique sont choisis plus particulièrement parmi les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à $10^{-13}$ et 1 à 40% en poids de ce polymère, d'un monomère anionique choisi parmi les acides acrylique ou méthacrylique, le poids moléculaire de ce polymère étant compris entre environ 50.000 et 10.000.000 déterminé par chromatographie par perméation de gel. De tels polymères sont décrits dans la demande EP-A-269.243.

Les polymères préférés sont entre autres les polymères comportant des groupements alkyle choisis parmi les groupements ayant 1 à 4 atomes de carbone et plus particulièrement des groupements méthyle, éthyle.

Parmi ces polymères, les copolymères de chlorure de diméthyldiallylammonium ou de diéthyldiallylammonium et d'acide acrylique sont particulièrement préférés.

A titre de produits particulièrement préférés, on peut citer le polymère vendu sous la dénomination "MERQUAT

280" par la Société CALGON sous forme d'une solution aqueuse à 35% de matière active, ce polymère étant un copolymère de chlorure de diallyldiméthylammonium et d'acte acrylique dans les proportions 80/20, la viscosité au viscosimètre Brookfield LVF module 4 étant comprise entre 4000 et 10.000 ops, le poids moléculaire étant environ égal à 1.300.000.

Les polymères substantifs plus particulièrement préférés conformément à l'invention sont choisis parmi :

a) les polymères de polyammonium quaternaire préparés et décrits dans le brevet français 2.270.846 constitués des motifs récurrents répondant à la formule :

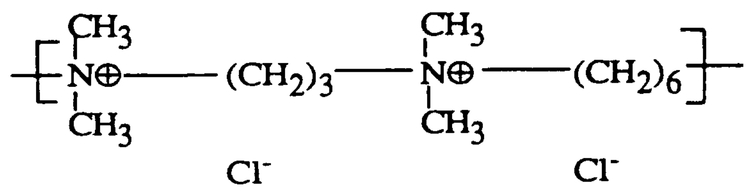

b) le copolymère de chlorure de diallyldiméthylammonium et d'acide acrylique (80/20) vendu sous la dénomination MERQUAT 280 par la Société CALGON;

c) l'homopolymère de chlorure de diméthyldiallylammonium vendu sous la dénomination MERQUAT 100 par la Société MERCK;

d) les dérivés d'éther de cellulose quaternisé vendus sous la dénomination JR par la Société UNION CARBIDE;

e) le copolymère de vinylpyrrolidone et de chlorure de méthacrylamidopropyltriméthylammonium (85/15) vendu sous la dénomination GAFQUAT HS 100 par la Société GAF.

Les compositions cosmétiques définies ci-dessus présentent une viscosité qui permet leur application notamment sur le cuir chevelu, cette viscosité n'augmente sensiblement pas au stockage. Elle présente une consistance de crème gélifiée ou de gel dans laquelle on peut introduire une quantité plus importante de matières premières ou d'adjuvants et notamment de matières actives, en particulier des colorants.

La composition conforme à l'invention est particulièrement intéressante dans son utilisation comme support de composition de teinture ou de décoloration des fibres kératinques, en particulier des cheveux.

On a pu constater que ces nouveaux supports, conformes à l'invention, présentent une plus grande facilité de mélange avec la solution oxydante, permettant des dilutions plus élevées sans perte notable de la consistance et des propriétés moussantes.

La composition contient dans cette forme de réalisation, des précurseurs de colorants d'oxydation connus en eux-mêmes, formant des colorants à la suite d'un processus de condensation oxydative de ces précurseurs de colorants, en présence éventuellement de couleurs ou modificateurs, ou des précurseurs indoliques générant des pigments de type mélanique sous l'action d'un oxydant et/ou des colorants directs.

Les précurseurs de colorants d'oxydation utilisables sont connus en eux-mêmes. On peut se référer plus particulièrement à ZVIAK, Science des Traitements capillaires 1988, pages 235 à 287. Il s'agit plus particulièrement de diamines ou aminophénols comportant des groupements fonctionnels amino et OH en position para ou ortho, les coupleurs ou modificateurs sont plus particulièrement des métadiamines, des méta-aminophénols ou des métadiphénols.

Ces compositions peuvent également contenir en plus des précurseurs de colorants d'oxydation, des colorants directs qui permettent d'enrichir les nuances tels que des colorants azoïques, anthraquinoniques, des dérivés nitrés de la série benzénique, des indamines, indoaniline et indophénols.

Les colorants indoliques générant des pigments du type mélanique sont plus particulièrement décrits dans les demandes de brevet et brevets français FR-A-2.593.061, 2.593.062, 2.595.245, 2.606.636, 2.636.237 et les demandes de brevet européen EP-A-425.345, EP-A-424.261. Les composés indoliques plus particulièrement préférés sont choisis parmi le 5,6-dihydroxyindole et ses dérivés et les 6- et 7-monohydroxyindoles.

Les compositions tinctoriales contenant le support conforme à l'invention, ne contiennent pas d'oxydant mais sont généralement utilisées conjointement avec une solution oxydante en vue de développer la coloration.

La demanderesse a constaté que grâce au nouveau support conforme à l'invention, le mélange avec la solution oxydante était beaucoup plus facile et permettait des dilutions plus élevées sans perte notable de la consistance et des propriétés moussantes de la composition.

Le milieu cosmétiquement acceptable des compositions conformes à l'invention est généralement un milieu aqueux ou un mélange d'eau avec un solvant organique miscible à l'eau. Parmi ces solvants, on peut citer les alcools

aliphatiques inférieurs tels que l'alcool éthylique, l'alcool propylique ou isopropylique, les glycols et éthers de glycols tels que le propylèneglycol, l'hexylèneglycol, le diéthylèneglycol, le dipropylèneglycol, les éthers monométhylique, - éthylique et - butylique de l'éthylèneglycol, l'éther monoéthylique du diéthylèneglycol, ainsi que leurs mélanges.

Les solvants particulièrement préférés sont l'alcool éthylique, le propylèneglycol, l'éther monobutylique de l'éthylèneglycol. Ces solvants peuvent être utilisés dans des proportions pondérales pouvant atteindre 20% en poids par rapport au poids total de la composition.

Les compositions conformes à l'invention peuvent également contenir les adjuvants tels que les agents alcalinisants, des agents conservateurs, des agents séquestrants, des parfums, des filtres solaires, des amies gras, des stérols naturels ou synthétiques, des acides gras en $C_{10}$-$C_{18}$, d'autres polymères différents des polymères substantifs conformes à l'invention ou des épaississants, sous réserve qu'ils ne déstabilisent pas le support.

La composition ne contient pas des alkylsulfates ou alkyléthersulfates susceptibles de déstabiliser la composition.

Les agents alcalinisants sont généralement utilisés dans des quantités suffisantes pour ajuster le pH à une valeur supérieure ou égale à 5,5. Ces agents alcalinisants sont choisis plus particulièrement parmi la soude, la potasse, l'ammoniaque, la monoéthanolamine, la diéthanolamine, la triéthanolamine, la mono- ou di-isopropanolamine, le 2-amino 2-méthyl 1,3-propanediol, le 2-amino 2-méthyl 1-propanol et leurs mélanges.

Les amides gras sont particulièrement choisis parmi le diéthanolamide oléique ou laurique, le mono-ou diéthanolamide de coprah, le monoéthanolamide d'acide d'alkyl($C_{13}$-$C_{15}$)éthercarboxylique oxyéthyléné à 2 moles d'oxyde d'éthylène, vendu sous la dénomination AMINOL A15 par la Société CHEM Y.

On peut également utiliser des polymères et des épaississants tels que plus particulièrement :

- l'ester monobutylique du copolymère méthylvinyléther/anhydride maléique, vendu sous la dénomination GANTREZ ES425 par la Société GAF;
- l'acide polyacrylamidométhylpropanesulfonique vendu sous la dénomination COSMEDIA POLYMER HSP 1180 par la Société HENKEL;
- le terpolymère acétate de vinyle/tert-butylbenzoate de vinyle/acide crotonique (65/25/10) tel que décrit dans le brevet français 2.439.798, neutralisé à 100% par l'amino-2 méthyl-2 propanol-1;
- des silices telles que la silice hydrophobe vendue sous la dénomination AEROSIL R 972 par la Société DEGUSSA ou la silice hydrophile vendue sous la dénomination SILICE HDK-N 20E par la Société WACKER CHEMIE;
- les acides polyacryliques réticulés tels que les CARBOPOLS vendus par la Société GOODRICH.

Pour la décoration des cheveux, on utilise les agents décolorants connus en eux-mêmes, tels que le peroxyde d'hydrogène, les persulfates, les percarbonates alcalins, les perborates.

De tels agents sont plus particulièrement décrits dans ZVIAK, Science des Traitements capillaires, 1988.

On utilise généralement en mettant en oeuvre du peroxyde d'hydrogène, des compositions à 60 volumes de peroxyde d'hydrogène et de préférence 10 à 40 volumes.

L'invention a également pour objet un procédé de teinture ou de décoloration des cheveux, caractérisé par le fait que l'on applique sur les cheveux en une quantité suffisante pour produire sort la teinture, soit la décoloration, une composition telle que définie ci-dessus, contenant le support conforme à l'invention.

Losqu'elle est utilisée pour la teinture, cette composition est généralement diluée au moment de l'emploi avec la solution oxydante dans un rapport allant de 0,5 à 5 et de préférence de 1 à 3 en volume. On laisse agir la composition pendant un temps compris entre 5 et 45 minutes environ et de préférence entre 15 et 30 minutes, puis on rince les cheveux.

Lorsqu'elle est utilisée pour la décoloration, elle ne contient pas de colorants ou de précurseurs et elle est directement appliquée sur les cheveux dans une quantité et pendant une durée suffisante pour décolorer les cheveux.

Les exemples suivants sont destinés à illustrer l'invention sans pour autant présenter un caractère limitatif.

EXEMPLE 1

| | | |
|---|---|---|
| - Alcool oléocétylique oxyéthyléné à 30 moles d'oxyde d'éthylène ($nC_A$=17 - HLB = 16,5) | 7 | g |
| - Alcool laurique($C_{12}$-$C_{14}$/55-45%) oxyéthyléné à 12 moles d'oxyde d'éthylène ($nC_A$=12,5 HLB = 14) | 8 | g |
| - Alcool cétylstéarylique($C_{16}$/$C_{18}$ - 50/50) ($nC_B$= 17-HLB=1) | 5 | g |
| - Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 - HLB = 8,5) | 22 | g |
| - Polymère cationique décrit et préparé selon le brevet français 2.270.846 constitué de motifs récurrents de formule : 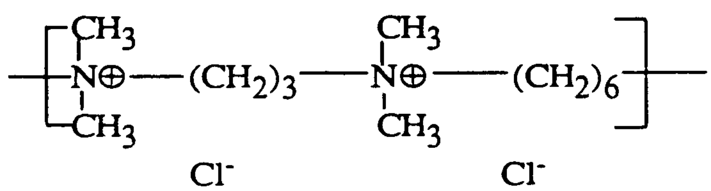 | 0,1 | g |
| - Propylèneglycol | 6 | g |
| - Solution aqueuse d'ammoniaque à 20% de $NH_3$ | 10 | g |
| - Paraphénylènediamine | 0,4 | g |
| - m-aminophénol | 0,5 | g |
| - Hydroquinone | 0,1 | g |
| - 1-phényl 3-méthyl 5-pyrazolone | 0,1 | g |
| - Solution aqueuse de bisulfite de sodium à 35% de MA | 1,3 | g |
| - Parfum, séquestrant qs | | |
| - Eau qsp | 100 | g |
| - pH =11,0 | | |

EXEMPLE 2

- Alcool oléocétylique oxyéthyléné à 30 moles d'oxyde d'éthylène ($nC_A$=17 - HLB = 16,5) 7 g
- Alcool laurique($C_{12}$-$C_{14}$/55-45%) oxyéthyléné à 12 moles d'oxyde d'éthylène ($nC_A$=12,5 - HLB = 14) 8 g
- Alcool cétylstéarylique ($C_{16}$/$C_{18}$-50/50) ($nC_B$=17 - HLB = 1) 5 g
- Alcool décylique($C_{10}$-$C_{12}C_{14}$/85-8,5-6,5)oxyéthyléné à 3,5 moles d'oxyde d'éthylène, vendu sous la dénomination MERGITAL BL 309 par la Société HENKEL ($nC_B$=10,4 - HLB=8,5) 22 g
- Polymère cationique décrit et préparé selon le brevet français 2.270.846 constitué de motifs récurrents de formule :

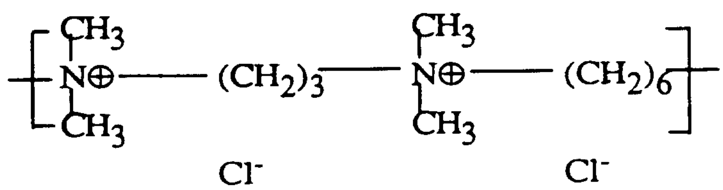

8 g

- Propylèneglycol 6 g
- Solution aqueuse d'ammoniaque à 20% de $NH_3$ 10 g
- Paraphénylènediamine 0,4 g
- m-aminophénol 0,5 g
- Hydroquinone 0,1 g
- 1-phényl 3-méthyl 5-pyrazolone 0,1 g
- Solution aqueuse de bisulfite de sodium à 35% de MA 1,3 g
- Parfum, séquestrant qs
- Eau qsp 100 g
- pH = 10,9

EXEMPLE 3

| | |
|---|---|
| - Alcool oléocétylique oxyéthyléné à 30 moles d'oxyde d'éthylène ($nC_A$=17 - HLB=16,5) | 7 g |
| - Alcool laurique($C_{12}$-$C_{14}$/55-45%)oxyéthyléné à 12 moles d'oxyde d'éthylène ($nC_A$=12,5 - HLB=14) | 8 g |
| - Alcool cétylstéarylique($C_{16}$/$C_{18}$-50/50)($nC_B$=17-HLB=1) | 5 g |
| - Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 - HLB=8,5) | 22 g |
| - Copolymère de chlorure de diallyl diméthyl ammonium et d'acide acrylique, vendu sous la dénomination MERQUAT 280 par la Société CALGON à 35% de MA | 3 g MA |
| - Acide polyacrylique réticulé vendu sous la dénomination CARBOPOL 934 (PM 3.000.000) par la Société GOODRICH | 0,4 g |
| - Propylèneglycol | 8 g |
| - Monoéthanolamine | 8,3 g |
| - Hydroquinone | 0,1 g |
| - 1-phényl 3-méthyl 5-pyrazolone | 0,1 g |
| - Solution aqueuse de bisulfite de sodium à 35% de MA | 1,3 g |
| - Paraphénylènediamine | 0,5 g |
| - m-dihydroxybenzène | 0,4 g |
| - Parfum, séquestrant qs | |
| - Eau qsp | 100 g |
| - pH = 11,0 | |

EXEMPLE 4

| | |
|---|---|
| - Alcool oléocétylique oxyéthyléné à 30 moles d'oxyde d'éthylène ($nC_A$=17 - HLB = 16,5) | 10,8 g |
| - Alcool laurique($C_{12}$-$C_{14}$/55-45%)oxyéthyléné à 12 moles d'oxyde d'éthylène ($nC_A$=12,5 - HLB = 14) | 2,5 g |
| - Alcool décylique($C_{10}$-$C_{12}$-$C_{14}$/85-8,5-6,5) oxyéthyléné à 5,5 moles d'oxyde d'éthylène, vendu sous la dénomination MERGITAL BL 589 par la Société HENKEL | 6,5 g |
| - Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 - HLB=8,5) | 18,5 g |
| - Alcool oléique (70% en $C_{18}$) ($nC_B$=17,5 - HLB = 1) | 2,25g |
| - Polymère d'hydroxyéthylcellulose et d'épichlorhydrine quaternisé avec la triméthylamine, vendu sous la dénomination JR 400 par la Société UNION CARBIDE | 0,5 g |
| - Propylèneglycol | 8 g |
| - Monoéthanolamine | 8 g |
| - Hydroquinone | 0,1 g |
| - 1-phényl 3-méthyl 5-pyrazolone | 0,1 g |
| - Solution aqueuse de bisulfite de sodium à 35% de MA | 1,3 g |
| - Paraphénylènediamine | 0,5 g |
| - m-dihydroxybenzène | 0,4 g |
| - Parfum, séquestrant qs | |
| - Eau qsp | 100 g |
| - pH = 11,0 | |

EXEMPLE 5

| | |
|---|---|
| - Alcool oléocétylique oxyéthyléné à 30 moles d'oxyde d'éthylène ($nC_A$=17 - HLB=16,5)) | 9 g |
| - Alcool laurique($C_{12}$-$C_{14}$/55-45%) oxyéthyléné à 12 moles d'oxyde d'éthylène ($nC_A$=12,5 - HLB=14) | 7 g |
| - Alcool décylique($C_{10}$-$C_{12}$-$C_{14}$/85-8,5-6,5) oxyéthyléné à 5,5 moles d'oxyde d'éthylène, vendu sous la dénomination MERGITAL BL 589 par la Société HENKEL | 12,2 g |
| - Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$ = 10,4 - HLB=8,5) | 8,8 g |
| - Alcool oléique (70% en $C_{18}$) ($nC_B$ = 17,5 - HLB=1) | 4 g |
| - Copolymère de vinylpyrrolidone et chlorure de méthacrylamidopropyl triméthyl ammonium (85/15), vendu en solution aqueuse à 20% de MA sous la dénomination GAFQUAT HS 100 par la Société GAF | 3 g |
| - Propylèneglycol | 8 g |
| - Monoéthanolamine | 8 g |
| - Hydroquinone | 0,1 g |
| - 1-phényl 3-méthyl 5-pyrazolone | 0,1 g |
| - Solution aqueuse de bisulfite de sodium à 35% de MA | 1,3 g |
| - Paraphénylènediamine | 0,5 g |
| - m-dihydroxybenzène | 0,4 g |
| - Parfum, séquestrant qs | |
| - Eau qsp | 100 g |
| - 10,9 pH | |

EXEMPLE 6

| | | |
|---|---|---|
| - Alcool oléocétylique oxyéthyléné à 30 moles d'oxyde d'éthylène ($nC_A$=17 - HLB=16,5) | 6 | g |
| - Alcool laurique($C_{12}$-$C_{14}$/55-45%) oxyéthyléné à 12 moles d'oxyde d'éthylène ($nC_A$=12,5- HLB=14) | 10 | g |
| - Alcool oléique polyglycérolé à 2 moles de glycérol ($nC_B$ = 18 - HLB=7,1) | 10 | g |
| - Alcool cétylstéarylique($C_{16}$/$C_{18}$-50/50) ($nC_B$ = 17-HLB=1) | 11 | g |
| - Polymère cationique décrit et préparé selon le brevet français 2.270.846 constitué de motifs récurrents de formule : 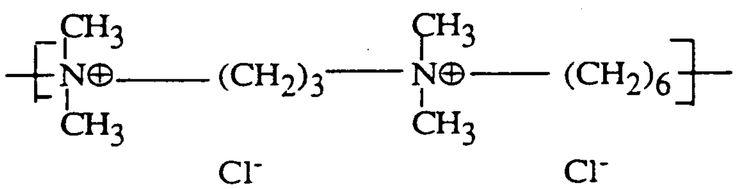 | 3 | g |
| - Propylèneglycol | 8 | g |
| - Triéthanolamine | 0,1 | g |
| - Hydroquinone | 0,1 | g |
| - 1-phényl 3-méthyl 5-pyrazolone | 0,1 | g |
| - Solution aqueuse de bisulfite de sodium à 35% de MA | 1,3 | g |
| - Paraphénylènediamine | 0,5 | g |
| - m-dihydroxybenzène | 0,4 | g |
| - Parfum, séquestrant qs | | |
| - Eau qsp | 100 | g |
| - pH = 6 | | |

EXEMPLE 7

| | | |
|---|---|---|
| - Alcool isostéarylique oxyéthyléné à 20 moles d'oxyde d'éthylène ($nC_A$=18 - HLB=14,9) | | 24 g |
| - Alcool isostéarylique oxyéthyléné à 2 moles d'oxyde d'éthylène ($nC_B$ = 18 - HLB=6,5) | | 20 g |
| - Polymère cationique décrit et préparé selon le brevet français 2.270.846 constitué de motifs récurrents de formule : 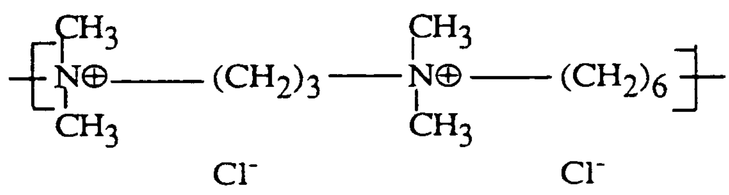 | | 3 g |
| - Propylèneglycol | | 20 g |
| - Monoéthanolamine | | 8 g |
| - Hydroquinone | | 0,1 g |
| - 1-phényl 3-méthyl 5-pyrazolone | | 0,1 g |
| - Solution aqueuse de bisulfite de sodium à 35% de MA | | 1,3 g |
| - Paraphénylènediamine | | 0,5 g |
| - m-dihydroxybenzène | | 0,4 g |
| - Parfum, séquestrant qs | | |
| - Eau | qsp | 100 g |
| - pH=10,9 | | |

EXEMPLE 8

- Alcool oléocétylique oxyéthyléné à 30 moles d'oxyde d'éthylène ($nC_A$=17 - HLB = 16,5) 6 g
- Alcool laurique($C_{12}$-$C_{14}$/55-45%)oxyéthyléné à 12 moles d'oxyde d'éthylène ($nC_A$=12,5 - HLB = 14) 10 g
- Alcool cétylstéarylique($C_{16}$/$C_{18}$-50/50) ($nC_B$ = 17 HLB = 1) 11 g
- Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$ = 10,4 - HLB = 8,5) 10 g
- Silice hydrophobe vendue sous la dénomination AEROSIL R 972 par la Société DEGUSSA 2 g
- Polymère cationique décrit et préparé selon le brevet français 2.270.846 constitué de motifs récurrents de formule :

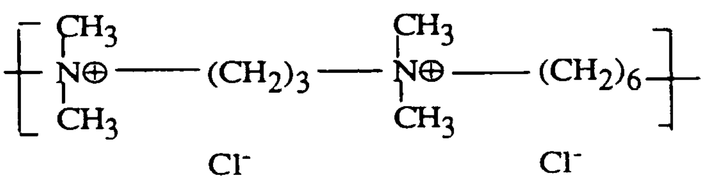

3 g

- Propylèneglycol 8 g
- Monoéthanolamine 8 g
- Acide laurique (neutralisé par 0,5 g de monoéthanolamine) 2 g
- Hydroquinone 0,1 g
- 1-phényl 3-méthyl 5-pyrazolone 0,1 g
- Solution aqueuse de bisulfite de sodium à 35% de MA 1,3 g
- Paraphénylènediamine 0,5 g
- m-dihydroxybenzène 0,4 g
- Parfum, séquestrant qs
- Eau qsp 100 g
- pH = 10,9

EXEMPLE 9

- Alcool oléocétylique oxyéthyléné à 30 moles d'oxyde d'éthylène ($nC_A$=17 - HLB = 16,5)) 3,9 g
- Alcool laurique($C_{12}$-$C_{14}$/55-45%) oxyéthyléné à 12 moles d'oxyde d'éthylène ($nC_A$=12,5 - HLB=14) 6,6 g
- Alcool décylique($C_{10}$-$C_{12}$-$C_{14}$/85-8,5-6,5) oxyéthyléné à 5,5 moles d'oxyde d'éthylène, vendu sous la dénomination MERGITAL BL 589 par la Société HENKEL 6,6 g
- Alcool décylique($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 - HLB=8,5) 22 g
- Alcool oléique (70% en $C_{18}$) ($nC_B$=17,5 - HLB=1) 1,1 g
- Polymère cationique décrit et préparé selon le brevet français 2.270.846 constitué de motifs récurrents de formule :

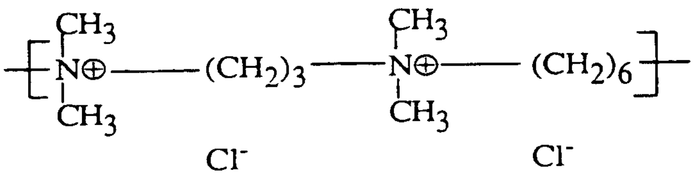

2,1 g

- Copolymère de chlorure de diallyl diméthyl ammonium et d'acide acrylique, vendu sous la dénomination MERQUAT 280 par la Société CALGON à 35% de MA 1,4 g MA
- Acide polyacrylique réticulé, vendu sous la dénomination CARBOPOL 934 (PM 3.000.000) par la Société GOODRICH 0,4 g
- Propylèneglycol 8 g
- Monoéthanolamine 8 g
- Hydroquinone 0,1 g
- 1-phényl 3-méthyl 5-pyrazolone 0,1 g
- Solution aqueuse de bisulfite de sodium à 35% de MA 1,3 g

| | | |
|---|---|---|
| - Paraphénylènediamine | | 0,5 g |
| - m-dihydroxybenzène | | 0,4 g |
| - Parfum, séquestrant | qs | |
| - Eau | qsp | 100 g |
| - pH = 11,0 | | |

EXEMPLE 10

| | |
|---|---|
| - Alcool isostéarylique oxyéthyléné à 20 moles d'oxyde d'éthylène $nC_A$ = 18 - HLB = 14,9 | 8 g |
| - Alcool isostéarylique oxyéthyléné à 2 moles d'oxyde d'éthylène $nC_B$ = 18 - HLB = 6,5 | 7 g |
| - Dérivé d'éther de cellulose quaternisé vendu par la société UNION CARBIDE sous la dénomination POLYMER JR 30 M | 2 g |
| - Propylèneglycol | 3 g |
| - Ammoniaque (20% de $NH_3$) | 5 g |
| - Hydroquinone | 0,1 g |
| - 1-phényl 3-méthyl 5-pyrazolone | 0,1 g |
| - Solution aqueuse de bisulfite de sodium à 35% de MA | 1,3 g |
| - Paraphénylènediamine | 0,5 g |
| - m-dihydroxybenzène | 0,4 g |
| - Parfum, séquestrant qs | |
| - Eau qsp | 100 g |
| - pH = 11 | |

Les compositions des exemples 1 à 10 sont stables au stockage.

Au moment de l'emploi, elles sont diluées avec un volume égal d'eau oxygénée à 20 volumes. On peut soit utiliser une solution aqueuse d'eau oxygénée à 20 volumes à pH = 3, soit une des deux émulsions oxydantes ci-après.

| a) | |
|---|---|
| - alcool cétylique | 1,5 g |
| - alcool cétylstéarylique oxyéthyléné à 15 moles d'oxyde d'éthylène | 2,5 g |
| - Sulfate d'ortho oxyquinoléine | 0,05 g |
| - Eau oxygénée à 200 vol. qsp | 20 vol |
| - HCl qsp pH 3 | |
| - Eau qsp | 100 g |
| b) | |
| - Alcool cétylstéarylique oxyéthyléné vendu sous la dénomination SINNOWAX par la Société HENKEL | 4 g |
| - Sulfate d'ortho oxyquinoléine | 0,05 g |
| - Eau oxygénée à 200 vol. qsp | 20 vol. |
| - HCl qsp pH 3 | |
| - Eau qsp | 100 g |

Appliquées sur cheveux décolorés pendant 30 minutes à température ambiante, les compositions des exemples 1 et 2 confèrent aux cheveux une couleur pourpre gris alors que les compositions des exemples 3 à 10 les colorent en châtain cendré.

EXEMPLE 11

| **Composition de coloration** | |
|---|---|
| - Alcool oléocétylique oxyéthyléné à 30 moles d'oxyde d'éthylène ($nC_A$=17 - HLB=16,5) | 4,2 g |
| - Alcool laurique ($C_{12}$-$C_{14}$/55-45%) oxyéthyléné à 12 moles d'oxyde d'éthylène ($nC_A$=12,5 -HLB=14) | 4,8 g |
| - Alcool cétylstéarylique ($C_{16}$/$C_{18}$-50/50) ($nC_B$=17 -HLB=1) | 3 g |
| - Alcool décylique ($C_{10}$-$C_{12}C_{14}$/85-8,5-6,5) oxyéthyléné à 3,5 moles d'oxyde d'éthylène, vendu sous la dénomination MERGITAL BL 309 par la Société HENKEL ($nC_B$=10,4 -HLB=8,5) | 13,2 g |
| - Homopolymère de chlorure de diallyldiméthylammonium, vendu sous la dénomination MERQUAT 100 par la Société MERCK à 40% de MA | 3 g MA |
| - Solution aqueuse d'ammoniaque à 20% de $NH_3$ | 12 g |
| - Paraphénylènediamine | 0,45 g |
| - m-dihydroxybenzène | 0,35 g |
| - Solution aqueuse de bisulfite de sodium à 35% de MA | 1,8 g |
| - Parfum, séquestrant qs | |
| - Eau qsp | 100 g |
| - pH = 10,9 | |

Appliquée sur cheveux blancs naturels, comme indiqué ci-dessus, la composition confère aux cheveux une coloration châtain.

EXEMPLE 12

| Composition de coloration | |
|---|---|
| - Acide oléique | 3 g |
| - Alcool oléocétylique oxyéthyléné à 30 moles d'oxyde d'éthylène ($nC_A$=17 - HLB=16,5) | 7 g |
| - Alcool laurique ($C_{12}$-$C_{14}$/55-45%) oxyéthyléné à 12 moles d'oxyde d'éthylène ($nC_A$=12,5 -HLB=14) | 8 g |
| - Alcool décylique ($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 -HLB=8,5) | 22 g |
| - Alcool oléique (70% en $C_{18}$) ($nC_B$=17,5 -HLB=1) | 5 g |
| - Homopolymère de chlorure de diallyldiméthylammonium, vendu sous la dénomination MERQUAT 100 par la Société MERCK à 40% de MA | 1,5 g MA |
| - Copolymère de chlorure de diallyldiméthylammonium et d'acide acrylique, vendu sous la dénomination MERQUAT 280 par la Société CALGON à 35% de MA | 1,5 g MA |
| - Paraphénylènediamine | 0,3 g |
| - m-aminophénol | 0,15 g |
| - Solution aqueuse de bisulfite de sodium à 35% de MA | 1,3 g |
| - Solution aqueuse d'ammoniaque à 20% d'$NH_3$ | 12 g |
| - Parfum, séquestrant qs | |
| - Eau qsp | 100 g |
| - pH = 10,9 | |

Appliquée sur cheveux blancs permanentés, comme indiqué ci-dessus, la coloration obtenue est pourpre gris foncé.

EXEMPLE 13

## Composition de décoloration

- Alcool oléocétylique oxyéthyléné à 30 moles d'oxyde d'éthylène ($nC_A$=17 - HLB=16,5) 7 g
- Alcool laurique ($C_{12}$-$C_{14}$/55-45%) oxyéthyléné à 12 moles d'oxyde d'éthylène ($nC_A$=12,5 - HLB=14) 8 g
- Alcool cétylstéarylique ($C_{16}$/$C_{18}$-50/50) ($nC_B$=17 - HLB=1) 5 g
- Alcool décylique ($C_{10}$-$C_{12}$-$C_{14}$/85-8,5-6,5) oxyéthyléné à 3,5 moles d'oxyde d'éthylène, vendu sous la dénomination MERGITAL BL 309 par la Société HENKEL ($nC_B$=10,4 - HLB=8,5) 22 g
- Polymère cationique décrit et préparé selon le brevet français 2.270.846 constitué de motifs récurrents de formule :

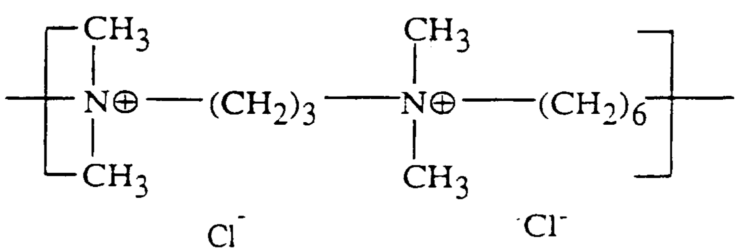

8 g

- Propylèneglycol 6 g
- Solution aqueuse d'ammoniaque à 20% de $NH_3$ 15 g
- Parfum, séquestrant qs
- Eau qsp 100 g
- pH = 10,9

### Revendications

**1.** Utilisation d'une composition contenant dans une milieu cosmétiquement acceptable :

a) 14 à 50% d'un mélange d'agents tensio-actifs non-ioniques choisis parmi les alcools gras, les alcools gras oxyéthylénés et/ou oxypropylénés et/ou polyglycérolés linéaires ou ramifiés, le mélange comprenant au moins un agent tensio-actif A dont le HLB au sens de Griffin est supérieur ou égal à 14 présent à une concentration pondérale [A] et d'un agent tensio-actif non-ionique B dont la valeur HLB au sens de Griffin est supérieure ou

égale à 1 et inférieure à 10, présent dans une quantité pondérale [B], plus de la moitié des agents tensio-actifs non-ioniques présents satisfaisant à l'inégalité :

$$0,5 \leq R \leq 1,6$$

dans laquelle R désigne le rapport

$$R = \frac{\Sigma(nC_A \times [A])}{\Sigma(nC_B \times [B])} = \frac{\text{Somme des produits } nC_A \times [A]}{\text{Somme des produits } nC_B \times [B]}$$

dans lequel :

$nC_A$ est le nombre d'atomes de carbone de la chaîne grasse du tensio-actif A et $nC_B$ le nombre d'atomes de carbone de la chaîne grasse de l'agent tensio-actif B;

b) 0,05 à 10%, d'un polymère substantif cationique ou amphotère,

la composition étant stable à température ambiante et a un pH supérieur ou égal à 5,5. Comme support dans de compositions, de teinture d'oxydation ou de décoloration.

**2.** Utilisation selon la revendication 1, caractérisée par le fait que les agents tensio-actifs sont présents dans la composition dans une proportion comprise entre 20 et 50%.

**3.** Utilisation selon la revendication 1 ou 2, caractérisée par le fait que les agents tensio-actifs non-ioniques présents dans la composition comportent moins de 48% d'agents tensio-actifs ne répondant pas à la définition des agents tensio-actifs A et B.

**4.** Utilisation selon l'une quelconque des revendications 1 à 3, caractérisée par le fait que les agents tensio-actifs non-ioniques autres que les agents tensio-actifs A et B, ont une valeur HLB comprise entre 10 et 14.

**5.** Utilisation selon l'une quelconque des revendications 1 à 4, caractérisée par le fait que les agents tensio-actifs ayant une valeur de HLB supérieure ou égale à 1 4 de type A, sont choisis parmi l'alcool oléocétylique oxyéthyléné à 30 moles d'oxyde d'éthyléne, l'alcool laurique ($C_{12}$-$C_{14}$/55- 45%) oxyéthyléné à 12 moles d'oxyde d'éthylène, l'alcool laurique oxyéthyléné à 23 moles d'oxyde d'éthylène, l'alcool stéarylique oxyéthyléne à 100 moles d'oxyde d'éthyléne, l'alcool isostéarylique oxyéthyléné à 20 moles d'oxyde d'éthylène, l'alcool béhénique oxyéthyléné à 20 moles d'oxyde d'éthylène, l'alcool primaire en $C_{30}$ oxyéthyléne à 40 moles d'oxyde d'éthylène, les octylphénols oxyéthylénés de 11 à 50 moles d'oxyde d'éthylène, les nonylphénols oxyéthylénés de 12 à 50 moles d'oxyde d'éthylène, l'alcool cétyl stéarylique oxyéthyléné à 15 moles d'oxyde d'éthylène..

**6.** Utilisation selon l'une quelconque des revendications 1 à 5, caractérisée par le fait que les agents tensio-actifs non-ioniques ayant une valeur HLB supérieure ou égale à 1 et inférieure ou égale à 10 de type B, sont choisis parmi l'alcool décylique oxyéthyléné à 3,5 moles d'oxyde d'éthyléne. l'alcool oléique à 70% de $C_{18}$, l'alcool laurique, l'alcool cétytstéarylique (50/50), l'alcool isostéarylique oxyéthyléné à 2 moles d'oxyde d'éthyléne, l'alcool oléique oxyéthyléné à 3 moles d'oxyde d'éthyléne, l'alcool laurique oxyéthyléné à 4 moles d'oxyde d'éthylène, l'alcool laurique oxypropyléné à 3 moles d'oxyde de propylène, l'alcool primaire en $C_{50}$ oxyéthyléné à 4 moles d'oxyde d'éthylène, l'alcool oléique polyglycérolé à 2 moles de glycérol, des octylphénols oxyéthylénés à moins de 4,5 moles d'oxyde d'éthylène, las nonylphénols oxyéthylénés à moins de 5 moles d'oxyde d'éthylène, l'acool primaire en $C_{50}$ oxyéthyléné à 4 moles d'oxyde d'éthyléne.

**7.** Utilisation selon l'une quelconque des revendications 1 a 6, catacterisée par le fait que les agents tensio-actifs non-ioniques autres que les agents tensio-actifs A et B, sont choisis parmi l'alcool décylique oxyethyléné à 5,5 moles d'oxyde d'éthyléne, le nonylphénol oxyéthyléné à 6 moles d'oxyde d'éthylène et l'alcool primaire en $C_{30}$ oxyéthyléné à 10 moles d'oxyde d'éthylène.

**8.** Utilisation selon l'une quelconque des revendications 1 à 7, caractérisée par le fait que les polymères substantifs sont choisis parmi les polymères comportant des groupements amine primaire, secondaire, tertiaire et/ou quaternaire faisant partie de la chaîne polymère ou directement reliés à celle-ci et ayant un poids moléculaire compris entre 500 et 5.000.000, et de préférence entre 1000 et 3.000.000.

**9.** Utilisation selon la revendication 8, caractérisée par le fait que les polymères substantifs sont choisis parmi les protéines quaternisées constituées par des polypeptides modifiés chimiquement et portant en bout de chaîne ou greffés sur celle-ci, des groupements ammonium quaternaires.

**10.** Utilisation selon la revendication 8, caractérisée par le fait que les polymères de la famille des polyamines, polyaminoamides, polyammonium quaternaires, sont choisis parmi

(1) les copolymères vinylpyrrolidone/acrylate ou méthacrylate de dialkylaminoalkyle quaternisés ou non;
(2) les dérivés d'éthers de cellulose comportant des groupements ammonium quaternaires;
(3) les dérivés de cellulose cationiques constitués par des copolymères de cellulose ou les dérivés de cellulose greffés par un monomère hydrosoluble d'ammonium quaternaire;
(4) les polysaccharides quaternisés;
(5) les polymères constitués de motifs pipérazinyle et de radicaux divalents alkyléne ou hydroxyalkylène à chaînes droites ou ramifiées, éventuellement interrompues par des atomes d'oxygéne, de soufre, d'azote ou par des cycles aromatiques ou hétérocycliques, ainsi que les produits d'oxydation et/ou de quaternisation de ces polymères;
(6) les polyaminoamides solubles dans l'eau, éventuellement réticulés et/ou alcoylés;
(7) les dérivés de polyaminoamides résultant de la condensation de polyalcoylène polyamines avec des acides polycarboxyliques suivie d'une alcoylation par des agents bifonctionnels;
(8) les polymères obtenus par réaction d'une polyalkylène polyamine comportant deux groupements amine primaire et au moins un groupement amine secondaire avec un acide dicarboxylique;
(9) les cyclopolymères à poids moléculaire de 20 000 à 3.000.000;
(10) les polymères de polyammonium quaternaires;
(11) les homopolymères ou copolymères dérivés d'esters ou d'amides acrylique ou méthacrylique;
(12) les polymères quaternaires de vinylpyrrolidone et de vinylimidazole;
(13) les polyamines;
(14) des polyalkyléne imines;
(15) des polymères contenant des motifs vinylpyridine ou vinylpyridinium;
(16) des condensats de polyamines et d'épichlorhydrines;
(17) les polyuréylènes;
(18) les dérivés de la chitine.

**11.** Utilisation selon la revendication 8, caractérisée par le fait que les polymères substantifs sont choisis parmi les polysiloxanes cationiques.

**12.** Utilisation selon l'une quelconque des revendications 1 à 7, caractérisée par le fait que les polymères substantifs sont choisis parmi les polymères amphotères.

**13.** Utilisation selon la revendication 12, caractérisée par le fait que le polymère amphotère est choisi parmi les dérivés du chitosane et parmi les polymères de diallyldialkylammonium et d'un monomère anionique.

**14.** Utilisation selon l'une quelconque des revendications 1 à 8, 10, 12 et 13 caractérisée par le fait que les polymères substantifs sont choisis parmi :

a) le polymère de polyammonium quaternaire constitué de motifs récurrents, répondent à le formule :

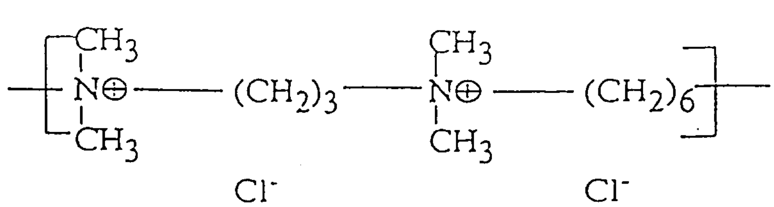

b) le copolymère de chlorure de diallyldiméthylammonium et d'acide acrylique;
c) l'homopolymère de chlorure de diméthyldiallylammonium;

d) les dérivés d'éther de cellulose quaternisés:

e) le copolymère de vinylpyrrolidone et de chlorure de méthacrylamidopropyltriméthylammonium.

**15.** Utilisation selon l'une quelconque des revendications 1 à 14, caractérisée par le fait qu'elle contient des adjuvants choisis parmi les agents alcalinisants, les agents conservateurs, les agents séquestrants, des parfums, des filtres solairés, des amides gras, des stérols naturels ou synthétiques, des acides gras en $C_{10}$-$C_{18}$, des polymères différents des polymères substantifs et des épaississants ne déstabilisant pas la composition.

**16.** Utilisation selon l'une quelconque des revendications 1 à 15, caractérisée par le fait que le milieu cosmétiquement acceptable est un milieu aqueux ou un mélange d'eau et d'un solvant organique miscible à l'eau.

**17.** Utilisation selon la revendication 16, caractérisée par le fait que les solvants sont choisis parmi les alcools aliphatiques inférieurs, les glycols et éthers de glycols, ainsi que leurs mélanges.

**18.** Composition de teinture des cheveux, caractérisée par le fait qu'elle contient dans une milieu cosmétiquement acceptable

a) 14 à 50% d'un mélange d'agents tensio-actifs non-ioniques choisis parmi les alcools gras, les alcools gras oxyéthylénés et/ou oxypropylénés et/ou polyglycérolés linéaires ou ramifiés, le mélange comprenant au moins un agent tensio-actif A dont le HLB au sens de Griffin est supérieur ou égal à 14 présent à une concentration pondérale [A] et d'un agent tensio-actif non-ionique B dont la valeur HLB au sens de Griffin est supérieure ou égale à 1 et inférieure à 10, présent dans une quantité pondérale [B], plus de la moitié des agents tensio-actifs non-ioniques présents satisfaisant à l'inégalité :

$$0{,}5 \leq R \leq 1{,}6$$

dans laquelle R désigne le rapport

$$R = \frac{\Sigma(nC_A \times [A])}{\Sigma(nC_B \times [B])} = \frac{\text{Somme des produits } nC_A \times [A]}{\text{Somme des produits } nC_B \times [B]}$$

dans lequel :

$nC_A$ est le nombre d'atomes de carbone de la chaîne grasse du tensio-actif et $nC_B$ le nombre d'atomes de carbonede la chaîne grasse de l'agent tensio-actif B;

b) 0,05 à 10% d'un polymère substantif cationique ou amphotère.
et un ou plusieurs précurseurs de colorants
d'oxydation et/ou un ou plusieurs colorants indoliques générant des pigments de type mélanique sous l'action d'un oxydant.
la composition étant stable à température ambiante et a un pH supérieur ou égal à 5,5.

**19.** Composition selon la revendication 18, caractérisée par le fait que la composition constituant le support est telle que définie dans l'une quelconque des revendications 2 à 17.

**20.** Composition selon la revendication 19, caractérisée par le fait que la composition contient également des colorants directs.

**21.** Composition de décoloration, caractérisée par le fait qu'elle contient dans une milieu cosmétiquement acceptable :

a) 14 à 50% d'un mélange d'agents tensio-actifs non-ioniques choisis parmi les alcools gras, les alcools gras oxyéthylénés et/ou oxypropylénés et/ou polyglycérolés linéaires ou ramifiés, le mélange comprenant au moins un agent tensio-actif A dont le HLB au sens de Griffin est supérieur ou égal à 14 présent à une concentration pondérale [A] et d'un agent tensio-actif non-ionique B dont la valeur HLB au sens de Griffin est supérieure ou égale à 1 et inférieure à 10, présent dans une quantité pondérale [B], plus de la moite des agents tensio-actifs non-ioniques présents satisfaisant à l'inégalité :

$$0{,}5 \leq R \leq 1{,}6$$

dans laquelle R désigne le rapport

$$R = \frac{\Sigma(nC_A \text{ x } [A])}{\Sigma(nC_B \text{ x } [B])} = \frac{\text{Somme des produits } nC_A \text{ x } [A]}{\text{Somme des produits } nC_B \text{ x } [B]}$$

dans lequel:

$nC_A$ est le nombre d'atomes de carbone de la chaîne grasse du tensio-actif A et $nC_B$ le nombre d'atomes de carbonede la chaîne grasse de l'agent tensio-actif B;

b) 0,05 à 10% d'un polymère substantif cationique ou amphotère,
et un agent décolorant les cheveux, ajouté au moment
la composition étant stable à température, ambiante et a un pH supérieur ou égal à 5,5,

**22.** Composition selon la revendication 21, caractérisée par le fait que la composition constituant le support est telle que définie dans l'une quelconque des revendications 2 à 17.

**23.** Composition selon la revendication 21, caractérisée par le fait que l'agent décolorant est choisi parmi le peroxyde d'hydrogène. les persulfates, les percarbonates de sodium, les perborates.

**24.** Procédé de coloration des cheveux, caractérisé par le fait que l'on applique sur ces cheveux, dans une quantité suffisante pour produire une coloration, la composition telle que définie dans l'une quelconque des revendications 19 ou 20, contenant un ou plusieurs précurseurs de colorants d'oxydation, diluée au moment de l'emploi avec une composition oxydante, qu'on laisse agir la composition pendant une durée comprise entre 5 et 45 minutes et qu'on rince les cheveux.

**25.** Procédé de décoloration des cheveux, caractérisé par le fait que l'on applique sur les cheveux la composition décolorante définie dans l'une quelconque des revendications 22 et 23, dans une quantité et pendant une durée suffisantes pour décolorer les cheveux et qu'on rince ensuite.

**Claims**

**1.** Use of a composition containing, in a cosmetically acceptable medium:

a) 14 to 50% of a mixture of nonionic surfactants chosen from fatty alcohols and oxyethylenated and/or oxypropylenated and/or polyglycerolated fatty alcohols which are linear or branched, the mixture comprising at least one surfactant A whose HLB in the sense used by Griffin is not less than 14, present at a weight concentration [A], and a nonionic surfactant B whose HLB value in the sense used by Griffin is not less than 1 and is less than 10, present in a weight quantity [B], more than half of the nonionic surfactants present satisfying the inequality:

$$0.5 \leq R \leq 1.6$$

in which R denotes the ratio

$$R = \frac{\Sigma(nC_A \times [A])}{\Sigma(nC_B \times [B])} = \frac{\text{Sum of the products } nC_A \times [A]}{\text{Sum of the products } nC_B \times [B]}$$

in which:

$nC_A$ is the number of carbon atoms in the fatty chain of the surfactant A and $nC_B$ the number of carbon atoms in the fatty chain of the surfactant B;

b) 0.05 to 10% of a cationic or amphoteric substantive polymer,
the composition being stable at room temperature and at a pH not less than 5.5,

as a support in oxidation dye compositions or bleaching compositions.

2. Use according to Claim 1, characterized in that the surfactants are present in the composition in a proportion of between 20 and 50%.

3. Use according to Claim 1 or 2, characterized in that the nonionic surfactants present in the composition contain less than 48% of surfactants not corresponding to the definition of the surfactants A and B.

4. Use according to any one of Claims 1 to 3, characterized in that the nonionic surfactants other than the surfactants A and B have an HLB value of between 10 and 14.

5. Use according to any one of Claims 1 to 4, characterized in that the type A surfactants having an HLB value of not less than 14 are chosen from oxyethylenated oleocetyl alcohol containing 30 mol of ethylene oxide, oxyethylenated lauryl alcohol ($C_{12}$-$C_{14}$/55-45%) containing 12 mol of ethylene oxide, oxyethylenated lauryl alcohol containing 23 mol of ethylene oxide, oxyethylenated stearyl alcohol containing 100 mol of ethylene oxide, oxyethylenated isostearyl alcohol containing 20 mol of ethylene oxide, oxyethylenated behenyl alcohol containing 20 mol of ethylene oxide, oxyethylenated $C_{30}$ primary alcohol containing 40 mol of ethylene oxide, oxyethylenated octylphenols containing 11 to 50 mol of ethylene oxide, oxyethylenated nonylphenols containing 12 to 50 mol of ethylene oxide and oxyethylenated cetyl/stearyl alcohol containing 15 mol of ethylene oxide.

6. Use according to any one of Claims 1 to 5, characterized in that the type B nonionic surfactants having an HLB value of not less than 1 and not more than 10 are chosen from oxyethylenated decyl alcohol containing 3.5 mol of ethylene oxide, oleyl alcohol containing 70% of $C_{18}$, lauryl alcohol, cetyl/stearyl (50:50) alcohol, oxyethylenated isostearyl alcohol containing 2 mol of ethylene oxide, oxyethylenated oleyl alcohol containing 3 mol of ethylene oxide, oxyethylenated lauryl alcohol containing 4 mol of ethylene oxide, oxypropylenated lauryl alcohol containing 3 mol of propylene oxide, oxyethylenated $C_{50}$ primary alcohol containing 4 mol of ethylene oxide, polyglycerolated oleyl alcohol containing 2 mol of glycerol, oxyethylenated octylphenols containing less than 4.5 mol of ethylene oxide, oxyethylenated nonylphenols containing less than 5 mol of ethylene oxide and oxyethylenated $C_{50}$ primary alcohol containing 4 mol of ethylene oxide.

7. Use according to any one of Claims 1 to 6, characterized in that the nonionic surfactants other than the surfactants A and B are chosen from oxyethylenated decyl alcohol containing 5.5 mol of ethylene oxide, oxyethylenated nonylphenol containing 6 mol of ethylene oxide and oxyethylenated $C_{30}$ primary alcohol containing 10 mol of ethylene oxide.

8. Use according to any one of Claims 1 to 7, characterized in that the substantive polymers are chosen from polymers containing primary, secondary, tertiary and/or quaternary amine groups, forming part of the polymer chain or linked directly to the latter, and having a molecular weight of between 500 and approximately 5,000,000, and preferably between 1000 and 3,000,000.

9. Use according to Claim 8, characterized in that the substantive polymers are chosen from quaternized proteins consisting of chemically modified polypeptides bearing quaternary ammonium groups at the end of the chain or grafted onto the latter.

10. Use according to Claim 8, characterized in that the polymers of the polyamine, polyaminoamide or poly(quaternary ammonium) family are chosen from

(1) vinylpyrrolidone/dialkylaminoalkyl acrylate or methacrylate copolymers, quaternized or otherwise;
(2) cellulose ether derivatives containing quaternary ammonium groups;
(3) cationic cellulose derivatives consisting of cellulose copolymers or cellulose derivatives grafted with a water-soluble quaternary ammonium monomer;
(4) quaternized polysaccharides;
(5) polymers consisting of piperazinyl units and divalent alkylene or hydroxyalkylene radicals having unbranched or branched chains optionally interrupted by oxygen, sulphur or nitrogen atoms or by aromatic or heterocyclic ring-systems, as well as the oxidation and/or quaternization products of these polymers;
(6) water-soluble polyaminoamides, optionally crosslinked and/or alkylated;
(7) polyaminoamide derivatives resulting from the condensation of polyalkylenepolyamines with polycarboxylic acids followed by an alkylation with bifunctional agents;

(8) polymers obtained by reaction of a polyalkylenepolyamine containing two primary amine groups and at least one secondary amine group with a dicarboxylic acid;

(9) cyclopolymers having a molecular weight of 20,000 to 3,000,000;

(10) poly(quaternary ammonium) polymers;

(11) homopolymers or copolymers derived from acrylic or methacrylic esters or amides;

(12) quaternary polymers of vinylpyrrolidone and of vinylimidazole;

(13) polyamines;

(14) polyalkylenimines;

(15) polymers containing vinylpyridine or vinylpyridinium units;

(16) condensates of polyamines and epichlorohydrins;

(17) polyureylenes;

(18) chitin derivatives.

**11.** Use according to Claim 8, characterized in that the substantive polymers are chosen from cationic polysiloxanes.

**12.** Use according to any one of Claims 1 to 7, characterized in that the substantive polymers are chosen from amphoteric polymers.

**13.** Use according to Claim 12, characterized in that the amphoteric polymer is chosen from chitosan derivatives and from polymers of diallyldialkylammonium and an anionic monomer.

**14.** Use according to any one of Claims 1 to 8, 10, 12 and 13, characterized in that the substantive polymers are chosen from:

a) the poly(quaternary ammonium) polymer consisting of recurring units corresponding to the formula:

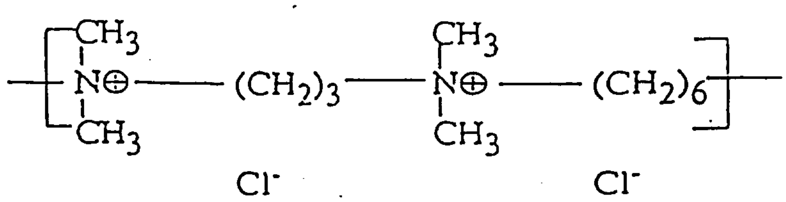

b) the copolymer of diallyldimethylammonium chloride and acrylic acid;

c) the homopolymer of dimethyldiallylammonium chloride;

d) quaternized cellulose ether derivatives;

e) the copolymer of vinylpyrrolidone and methacrylamidopropyltrimethylammonium chloride.

**15.** Use according to any one of Claims 1 to 14, characterized in that it contains adjuvants chosen from alkalinizing agents, preservatives, sequestering agents, perfumes, sunscreen agents, fatty amides, natural or synthetic sterols, $C_{10}$-$C_{18}$ fatty acids, polymers other than the substantive polymers and thickeners that do not destabilize the composition.

**16.** Use according to any one of Claims 1 to 15, characterized in that the cosmetically acceptable medium is an aqueous medium or a mixture of water and a water-miscible organic solvent.

**17.** Use according to Claim 16, characterized in that the solvents are chosen from lower aliphatic alcohols, glycols and glycol ethers as well as mixtures thereof.

**18.** Hair dyeing composition, characterized in that it contains, in a cosmetically acceptable medium:

a) 14 to 50% of a mixture of nonionic surfactants chosen from fatty alcohols and oxyethylenated and/or oxypropylenated and/or polyglycerolated fatty alcohols which are linear or branched, the mixture comprising at least one surfactant A whose HLB in the sense used by Griffin is not less than 14, present at a weight concentration [A], and a nonionic surfactant B whose HLB value in the sense used by Griffin is not less than 1 and is less than 10, present in a weight quantity [B], more than half of the nonionic surfactants present satisfying the inequality:

$$0.5 \leq R \leq 1.6$$

in which R denotes the ratio

$$R = \frac{\Sigma(nC_A \times [A])}{\Sigma(nC_B \times [B])} = \frac{\text{Sum of the products } nC_A \times [A]}{\text{Sum of the products } nC_B \times [B]}$$

in which:

$nC_A$ is the number of carbon atoms in the fatty chain of the surfactant A and $nC_B$ the number of carbon atoms in the fatty chain of the surfactant B;

b) 0.05 to 10% of a cationic or amphoteric substantive polymer,
and one or more oxidation dye precursors and/or one or more indole dyes which generate melanin-type pigments under the action of an oxidizing agent,
the composition being stable at room temperature and at a pH not less than 5.5.

**19.** Composition according to Claim 18, characterized in that the composition constituting the support is as defined in any one of Claims 2 to 17.

**20.** Composition according to Claim 19, characterized in that the composition also contains direct dyes.

**21.** Bleaching composition, characterized in that it contains, in a cosmetically acceptable medium:

a) 14 to 50% of a mixture of nonionic surfactants chosen from fatty alcohols and oxyethylenated and/or oxypropylenated and/or polyglycerolated fatty alcohols which are linear or branched, the mixture comprising at least one surfactant A whose HLB in the sense used by Griffin is not less than 14, present at a weight concentration [A], and a nonionic surfactant B whose HLB value in the sense used by Griffin is not less than 1 and is less than 10, present in a weight quantity [B], more than half of the nonionic surfactants present satisfying the inequality:

$$0.5 \leq R \leq 1.6$$

in which R denotes the ratio

$$R = \frac{\Sigma(nC_A \times [A])}{\Sigma(nC_B \times [B])} = \frac{\text{Sum of the products } nC_A \times [A]}{\text{Sum of the products } nC_B \times [B]}$$

in which:

$nC_A$ is the number of carbon atoms in the fatty chain of the surfactant A and $nC_B$ the number of carbon atoms in the fatty chain of the surfactant B;

b) 0.05 to 10% of a cationic or amphoteric substantive polymer,
and a hair bleaching agent, added at the moment [lacuna],
the composition being stable at room temperature and at a pH not less than 5.5.

**22.** Composition according to Claim 21, characterized in that the composition constituting the support is as defined in any one of Claims 2 to 17.

**23.** Use according to Claim 21, characterized in that the bleaching agent is chosen from hydrogen peroxide, persulphates, perborates and sodium percarbonates.

**24.** Hair dyeing process, characterized in that the composition as defined in either of Claims 19 and 20, containing one or more oxidation dye precursors and diluted at the time of use with an oxidizing composition, is applied to this hair in a sufficient quantity to produce a coloration, that the composition is allowed to act for a period of between 5 and 45 minutes approximately, and that the hair is rinsed.

25. Hair bleaching process, characterized in that the bleaching composition defined in either of Claims 22 and 23 is applied to the hair in a sufficient quantity and for a sufficient period to bleach the hair, and that the hair is then rinsed.

## Patentansprüche

1. Verwendung einer Zusammensetzung, enthaltend in einem kosmetisch geeigneten Milieu:

a) 14 bis 50% einer Mischung aus nicht-ionischen oberflächenaktiven Mitteln, ausgewählt aus Fettalkoholen, oxethylierten und/oder oxpropylierten und/oder polyglycerierten linearen oder verzweigten Fettalkoholen, wobei die Mischung mindestens ein oberflächenaktives Mittel A mit einem HLB-Wert gemäß Griffin von mehr als oder gleich 14, wobei dieses oberflächenaktive Mittel in einer Gewichtsmenge gleich (A) vorhanden ist, sowie mindestens ein nichtionisches oberflächenaktives Mittel B enthält, das einen HLB-Wert gemäß Griffin von mehr als oder gleich 1 und von weniger als 10 aufweist und in einer Gewichtsmenge (B) vorhanden ist, wobei mehr als die Hälfte der vorhandenen nicht-ionischen oberflächenaktiven Mittel die Ungleichheitsbedingung erfüllen:

$$0{,}5 \leq R \leq 1{,}6,$$

worin die Beziehung gilt:

$$R = \frac{\Sigma(nC_A x(A))}{\Sigma(nC_B x(B))} = \frac{\text{Summe des Produkts } nC_A x(A}{\text{Summe des Produkts } nC_B x(B)}$$

worin gilt:

$nC_A$ und $nC_B$ bedeuten jeweils die Anzahl an Kohlenstoffatomen der Fettkette der oberflächenaktiven Mittel A und B;

b) 0,05 bis 10% eines kationischen oder amphoteren substantiven Polymer,
wobei die genannte Zusammensetzung bei Umgebungstemperatur stabil ist und einen pH-Wert von gleich oder mehr als 5,5 aufweist,
als Trägermedium in Zusammensetzungen zur Oxidationsfärbung oder Entfärbung.

2. Verwendung gemäß Anspruch 1,
dadurch **gekennzeichnet**, daß
die oberflächenaktiven Mittel in der Zusammensetzung in einem Mengenanteil von 20 bis 50% vorhanden sind.

3. Verwendung gemäß Anspruch 1 oder 2,
dadurch **gekennzeichnet,** daß
die in der Zusammensetzung vorhandenen nicht-ionischen oberflächenaktiven Mittel weniger als 48% oberflächenaktive Mittel enthalten, die nicht die Definition der oberflächenaktiven Mittel A und B erfüllen.

4. Verwendung gemäß einem der Ansprüche 1 bis 3,
dadurch **gekennzeichnet**, daß
die nicht-ionischen oberflächenaktiven Mittel, die sich von den oberflächenaktiven Mitteln A und B unterscheiden, einen HLB-Wert von 10 bis 14 aufweisen.

5. Verwendung gemäß einem der Ansprüche 1 bis 4,
dadurch **gekennzeichnet,** daß
die oberflächenaktiven Mittel mit einem HLB-Wert von mehr als oder gleich 14 des Typs A ausgewählt sind aus Oleocetylalkohol, oxethyliert mit 30 Mol Ethylenoxid, Lauryl($C_{12-14}$/55-45%)alkohol, oxethyliert mit 12 Mol Ethylenoxid, Laurylalkohol, oxethyliert mit 23 Mol Ethylenoxid, Stearylalkohol, oxethyliert mit 100 Mol Ethylenoxid, Isostearylalkohol, oxethyliert mit 20 Mol Ethylenoxid, Behenylalkohol, oxethyliert mit 20 Mol Ethylenoxid, primärem $C_{30}$-Alkohol, oxethyliert mit 40 Mol Ethylenoxid, Octylphenolen, oxethyliert mit 11 bis 50 Mol Ethylenoxid, Nonylphenolen, oxethyliert mit 12 bis 50 Mol Ethylenoxid, und aus Cetylstearylalkohol, oxethyliert mit 15 Mol Ethylenoxid.

6. Verwendung gemäß einem der Ansprüche 1 bis 5,
dadurch **gekennzeichnet,** daß
die nicht-ionischen oberflcähenaktiven Mittel mit einem HLB-Wert von mehr als oder gleich 1 und weniger als oder gleich 10 des Typs B ausgewählt sind aus Decylalkohol, oxethyliert mit 3,5 Mol Ethylenoxid, Oleylalkohol mit 70% an $C_{18}$, Laurylalkohol, Cetylstearylalkohol (50/50), Isostearylalkohol, oxethyliert mit 2 Mol Ethylenoxid, Oleylalkohol, oxethyliert mit 3 Mol Ethylenoxid, Laurylalkohol, oxethyliert mit 4 Mol Ethylenoxid, Laurylalkohol, oxpropyliert mit 3 Mol Propylenoxid, primärem $C_{50}$-Alkohol, oxethyliert mit 4 Mol Ethylenoxid, Oleylalkohol, polyglyceriert mit 2 Mol Glycerin, Octylphenolen, oxethyliert mit mindestens 4,5 Mol Ethylenoxid, Nonylphenolen, oxethyliert mit mindestens 5 Mol Ethylenoxid, und aus primärem $C_{50}$-Alkohol, oxethyliert mit 4 Mol Ethylenoxid.

7. Verwendung gemäß einem der Ansprüche 1 bis 6,
dadurch **gekennzeichnet**, daß
die nicht-ionischen oberflächenaktiven Mittel, die sich von den oberflächenaktiven Mitteln A und B unterscheiden, aus mit 5,5 Mol Ethylenoxid oxethyliertem Decylalkohol, mit 6 Mol Ethylenoxid oxethyliertem Nonylphenol und aus mit 10 Mol Ethylenoxid oxethyliertem primären $C_{30}$-Alkohol ausgewählt sind.

8. Verwendung gemäß einem der Ansprüche 1 bis 7,
dadurch **gekennzeichnet**, daß
die substantiven Polymeren aus Polymeren ausgewählt sind, die primäre, sekundäre, tertiäre und/oder quaternäre Amingruppen, die Teil der Polymerkette oder direkt an diese gebunden sind, aufweisen und ein Molekulargewicht von 500 bis 5.000.000 und vorzugsweise von 1000 bis 3.000.000 aufweisen.

9. Verwendung gemäß Anspruch 8,
dadurch **gekennzeichnet**, daß
die substantiven Polymeren aus quaternierten Proteinen aus Polypeptiden ausgewählt sind, die chemisch modifiziert sind und am Ende der Kette oder auf diese gepfropft quaternäre Ammoniumgruppen aufweisen.

10. Verwendung gemäß Anspruch 8,
dadurch **gekennzeichnet**, daß
die Polymeren der Familie der quaternären Polyamine, Polyaminoamide oder Polyammonium-Verbindungen ausgewählt sind aus:

(1) Copolymeren aus Vinylpyrrolidon/Dialkylaminoalkylacrylat oder -methacrylat, quaterniert oder nicht;
(2) Derivaten von Celluloseethern, die quaternäre Ammoniumgruppen aufweisen;
(3) kationischen Cellulosederivaten aus Copolymeren von Cellulose oder aus Cellulosederivaten, die mit einem wasserlöslichen Monomer einer quaternären Ammoniumverbindung gepfropft sind;
(4) quaternierten Polysacchariden;
(5) Polymeren aus Piperazinyl-Einheiten und zweiwertigen Alkylen- oder Hydroxyalkylenresten mit geraden oder verzweigten Ketten, die gegebenenfalls mit Sauerstoff-, Schwefel-, Stickstoffatomen oder mit aromatischen oder heterozyklischen Ringen unterbrochen sind, sowie aus den Oxidations- und/oder Quaternierungsprodukten dieser Polymeren;
(6) wasserlöslichen Polyaminoamiden, die gegebenenfalls vernetzt und/oder alkyliert sind;
(7) Derivaten von Polyaminoamiden aus der Kondensation von Polyalkylenpolyaminen mit Polycarboxylsäuren unter anschließender Alkylierung mit bifunktionellen Mitteln;
(8) Polymeren aus der Reaktion eines Polyalkylenpolyamids mit zwei primären Amingruppen und mindestens einer sekundären Amingruppe mit einer Dicarboxylsäure;
(9) Cyclopolymeren mit einem Molekulargewicht von 20000 bis 3.000.000;
(10) Polymeren aus quaternären Polyammonium-Verbindungen;
(11) Homopolymeren oder Copolymeren aus Acryl- oder Methacrylestern oder -amiden;
(12) quaternären Polymeren aus Vinylpyrrolidon und Vinylimidazol;
(13) Polyaminen;
(14) Polyalkyleniminen;
(15) Polymeren mit Vinylpyridin- oder Vinylpyridinium-Einheiten
(16) Kondensaten aus Polyaminen und Epichlorhydrinen;
(17) Polyharnstoffen;
(18) Derivaten des Chitins.

11. Verwendung gemäß Anspruch 8,

dadurch **gekennzeichnet**, daß die substantiven Polymeren aus kationoschen Polysiloxanen ausgewählt sind.

**12.** Verwendung gemäß einem der Ansprüche 1 bis 7, dadurch **gekennzeichnet**, daß die substantiven Polymeren aus amphoteren Polymeren ausgewählt sind.

**13.** Verwendung gemäß Anspruch 12, dadurch **gekennzeichnet**, daß das amphotere Polymer aus Derivaten des Chitosans und aus Polymeren einer Diallyldialkylammonium-Verbindung und einem anionischen Monomer ausgewählt ist.

**14.** Verwendung gemäß einem der Ansprüche 1 bis 8, 10, 12 und 13, dadurch **gekennzeichnet**, daß die substantiven Polymeren ausgewählt sind aus:

a) dem Polymer einer quaternären Polyammonium-Verbindung aus wiederkehrenden Einheiten der Formel:

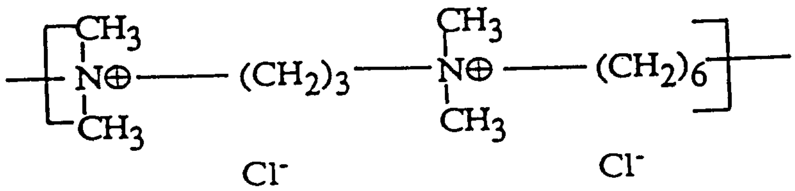

b) dem Copolymer aus Diallyldimethylammoniumchlorid und Acrylsäure;
c) dem Homopolymer aus Dimethyldiallyldiammoniumchlorid;
d) quaternierten Celluloseetherderivaten;
e) dem Copolymer aus Vinylpyrrolidon und Methacrylamidopropyltrimethylammoniumchlorid.

**15.** Verwendung gemäß einem der Ansprüche 1 bis 14, dadurch **gekennzeichnet**, daß sie Hilfsstoffe enthält, ausgewählt aus alkalisch stellenden Mitteln, Konservierungsmitteln, Sequestriermitteln, Parfüm-Produkten, Sonnenfilterstoffen, Fettamiden, natürlichen oder synthetischen Styrolen, $C_{10-18}$-Fettsäuren, Polymeren, die sich von den substantiven Polymeren unterscheiden, und aus Verdickungsmitteln, die die Zusammensetzung nicht destabilisieren.

**16.** Verwendung gemäß einem der Ansprüche 1 bis 15, dadurch **gekennzeichnet**, daß das kosmetisch geeignete Milieu ein wässriges Milieu oder eine Mischung aus Wasser und einem wasserlöslichen organischen Lösungsmittel ist.

**17.** Verwendung gemäß Anspruch 16, dadurch **gekennzeichnet**, daß die Lösungsmittel aus aliphatischen Niedrigalkoholen, Glycolen und Ethern von Glycolen sowie aus deren Mischungen ausgewählt sind.

**18.** Zusammensetzung zur Färbung der Haare, dadurch **gekennzeichnet**, daß sie in einem kosmetisch geeigneten Milieu enthält:

a) 14 bis 50% einer Mischung aus nicht-ionischen oberflächenaktiven Mitteln, ausgewählt aus Fettalkoholen, oxethylierten und/oder oxpropylierten und/oder polyglycerierten linearen oder verzweigten Fettalkoholen, wobei die Mischung mindestens ein oberflächenaktives Mittel A mit einem HLB-Wert gemäß Griffin von mehr als oder gleich 14, wobei dieses oberflächenaktive Mittel in einer Gewichtsmenge gleich (A) vorhanden ist, sowie mindestens ein nichtionisches oberflächenaktives Mittel B enthält, das einen HLB-Wert gemäß Griffin

von mehr als oder gleich 1 und von weniger als 10 aufweist und in einer Gewichtsmenge (B) vorhanden ist, wobei mehr als die Hälfte der vorhandenen nicht-ionischen oberflächenaktiven Mittel die Ungleichheitsbedingung erfüllen:

$$0{,}5 \leq R \leq 1{,}6,$$

worin die Beziehung gilt:

$$R = \frac{\Sigma(nC_A x(A))}{\Sigma(nC_B x(B))} = \frac{\text{Summe des Produkts } nC_A x(A}{\text{Summe des Produkts } nC_B x(B)}$$

worin gilt:

$nC_A$ und $nC_B$ bedeuten jeweils die Anzahl an Kohlenstoffatomen der Fettkette der oberflächenaktiven Mittel A und B;

b) 0,05 bis 10% eines kationischen oder amphoteren substantiven Polymer,
und eine oder mehrere Oxidationsfarbstoff-Vorstufenverbindungen und/oder einen oder mehrere indolischer Farbstoffe, die unter Beeinwirkung eines oxidierenden Mittels Pigmente vom Melanin-Typ erzeugen,
wobei die Zusammensetzung bei Umgebungstemperatur stabil ist und einen pH-Wert von gleich oder mehr als 5,5 aufweist.

**19.** Zusammensetzung gemäß Anspruch 18,
dadurch **gekennzeichnet**, daß
die Zusammensetzung, die das Trägermedium darstellt, die in jedem der Ansprüche 2 bis 17 definierte ist.

**20.** Zusammensetzung gemäß Anspruch 19,
dadurch **gekennzeichnet**, daß
die Zusammensetzung auch Direktfarbstoffe enthält.

**21.** Zusammensetzung zur Entfärbung,
dadurch **gekennzeichnet**, daß
sie in einem kosmetisch geeigneten Milieu enthält:

a) 14 bis 50% einer Mischung aus nicht-ionischen oberflächenaktiven Mitteln, ausgewählt aus Fettalkoholen, oxethylierten und/oder oxpropylierten und/oder polyglycerierten linearen oder verzweigten Fettalkoholen, wobei die Mischung mindestens ein oberflächenaktives Mittel A mit einem HLB-Wert gemäß Griffin von mehr als oder gleich 14, wobei dieses oberflächenaktive Mittel in einer Gewichtsmenge gleich (A) vorhanden ist, sowie mindestens ein nichtionisches oberflächenaktives Mittel B enthält, das einen HLB-Wert gemäß Griffin von mehr als oder gleich 1 und von weniger als 10 aufweist und in einer Gewichtsmenge (B) vorhanden ist, wobei mehr als die Hälfte der vorhandenen nicht-ionischen oberflächenaktiven Mittel die Ungleichheitsbedingung erfüllen:

$$0{,}5 \leq R \leq 1{,}6,$$

worin die Beziehung gilt:

$$R = \frac{\Sigma(nC_A x(A))}{\Sigma(nC_B x(B))} = \frac{\text{Summe des Produkts } nC_A x(A}{\text{Summe des Produkts } nC_B x(B)}$$

worin gilt:

$nC_A$ und $nC_B$ bedeuten jeweils die Anzahl an Kohlenstoffatomen der Fettkette der oberflächenaktiven Mittel A und B;

b) 0,05 bis 10% eines kationischen oder amphoteren substantiven Polymer,

und ein Entfärbungsmittel für die Haare, das zum Zeitpunkt der Anwendung zugefügt wird, wobei die Zusammensetzung bei Umgebungstemperatur stabil ist und einen pH-Wert von gleich oder mehr als 5,5 aufweist.

**22.** Zusammensetzung gemäß Anspruch 21,
dadurch **gekennzeichnet**, daß
die Zusammensetzung, die das Trägermedium darstellt, die in jedem der Ansprüche 2 bis 17 definierte ist.

**23.** Zusammensetzung gemäß Anspruch 21,
dadurch **gekennzeichnet**, daß
das entfärbende Mittel aus Wasserstoffperoxid, Persulfaten, Natriumpercarbonaten und aus Perboraten ausgewählt ist.

**24.** Verfahren zur Färbung der Haare,
dadurch **gekennzeichnet**, daß
man auf diese Haare in einer zur Erzeugung der Färbung ausreichenden Menge die in einem der Ansprüche 19 oder 20 definierte Zusammensetzung aufbringt, die mindestens einen oder mehrere Oxidationsfarbstoff-Vorstufenverbindungen enthält, welche zum Zeitpunkt der Anwendung mit einer oxidierenden Zusammensetzung verdünnt wird, daß man die Zusammensetzung während einer Zeitdauer von 5 bis 45 Minuten einwirken läßt und die Haare dann spült.

**25.** Verfahren zur Entfärbung der Haare,
dadurch **gekennzeichnet**, daß
man auf die Haare die in einem der Ansprüche 22 und 23 definierte entfärbende Zusammensetzung in einer Menge und während einer Zeitdauer aufbringt, die zur Entfärbung der Haare ausreichen, und daß man dann spült.